# EUROPEAN PATENT SPECIFICATION

(11) **EP 3 924 026 B1**
(45) Date of publication and mention of the grant of the patent: **07.10.2026**
(21) Application number: 20755288.6
(22) Date of filing: 13.02.2020
(51) Int. Cl.: A61M 16/04, A61M 16/00

(54) **CATHETER INFLATABLE CUFF PRESSURE-SENSING DEVICES**
DRUCKMESSUNGSVORRICHTUNGEN FÜR AUFBLASBARE KATHETERMANSCHETTEN
DISPOSITIF DE CAPTEUR DE PRESSION POUR BALLONNET GONFLABLE DE CATHÉTER

(30) Priority: 14.02.2019 WO PCT/IL2019/050176; 31.05.2019 US 201962855061 P; 21.08.2019 US 201962889804 P
(43) Date of publication of application: 22.12.2021
(73) Proprietor: Airway Medix S.A., 00-420 Warszawa (PL)
(72) Inventor: RAMOT, Yair, 4992500 Kfar Maas (IL); AMAR, Eizik, 7751344 Ashdod (IL); ZACHAR, Oron, 65153 Tel Aviv (IL)
(74) Representative: Patpol Kancelaria Patentowa Sp. z o.o.
(86) International application number: PCT/IL2020/050166
(87) International publication number: WO 2020/165903

(56) References cited:
- EP-B1- 1 042 028
- EP-B1- 2 968 817
- WO-A1-2017/153988
- WO-A2-2009/037447
- WO-A2-2014/145411
- US-A1- 2014 261 442
- US-A1- 2015 283 343
- US-A1- 2018 093 057

## Description

### CROSS-REFERENCE TO RELATED APPLICATIONS

The present application (a) claims priority from and is a continuation-in-part of International Application PCT/IL2019/050176, filed February 14, 2019, which published as PCT Publication WO 2019/162939, and (b) claims priority from (i) US Provisional Application 62/855,061, filed May 31, 2019, and (ii) US Provisional Application 62/889,804, filed August 21, 2019. All of the above-mentioned applications are assigned to the assignee of the present application.

### FIELD OF THE APPLICATION

The present invention relates generally to medical suction catheter systems, and specifically to airway ventilation device cuff systems as defined by the appended claims.

### BACKGROUND OF THE APPLICATION

Some endotracheal tubes (ETTs) comprise an inflatable cuff, which forms a seal against the tracheal wall. This seal prevents gases from leaking past the cuff and allows positive pressure ventilation. Desired safe inflatable cuff pressure is in the range of 23 - 27 cm H2O, with an optimal pressure of about 25 cm H2O. Pressure above 30 cm H2O can cause irritation to the surrounding tracheal tissue. Extended duration of such high cuff pressure can interfere with oxygen flow to the tissue, causing tissue necrosis and a substantial wound. Low cuff pressure, typically below 20 cm H2O, compromises the cuff sealing performance, and allows leakage into the lungs of subglottic fluids descending from above the cuff.

The external surface of inflatable cuffs is in communication with the ventilation pressure of the lungs. The pressure of the inflatable cuff cycles with the ventilation cycle. When an artificially-ventilated patient is also anesthetized, the plastic of the inflatable cuff absorbs the nitrous oxide (N2O) gas used in anesthesia, which increases pressure in the cuff.

In current clinical settings of intensive care patients, changes of body positioning lead to significant changes in cuff pressure in the range of 10 - 50 cm H2O, i.e., out of the safe range of 20-30 cm H2O, and certainly out of the desired range of 23-27 cm H2O. See, for example, Lizy C et al., "Cuff pressure of endotracheal tubes after changes in body position in critically ill patients treated with mechanical ventilation," Am J Crit Care. 2014 Jan;23(1):e1-8.

Therefore, there is a need to safely maintain the inflatable cuff pressure is in the range of 23 - 27 cm H2O, optimally about 25 cm H2O, and to avoid extended periods of pressure above 30 cm H2O. In particular, there is a need to suppress the fluctuations of pressure in clinical settings caused by patient change of body positions.

Currently, the most common practiced approach for ETT cuff pressure management is manual monitoring (using a manometer) and adjustment of cuff pressure, which contributes to ICU staff workload. It has been shown that up to eight manual adjustments of cuff pressure are required daily to maintain recommended cuff pressure ranges. Even so, the cuff pressure is uncontrolled during the long time periods between manual cuff adjustments. In addition, the manometer must be connected to and disconnected from the ETT cuff for each pressure measurement, which allows a small amount of air to escape from the ETT cuff. Still further, many conventional ETT manometers lose calibration relatively quickly.

Prior art cuff pressure regulators can be divided into two groups: (a) large bedside non-disposable expensive electric pump and electronic pressure monitors; and (b) small and light disposable non-electric limited-pressure reservoir compartments that must be filled manually. Use of disposable devices both prevents cross-contamination between patients and obviates the need for costly sterilization processes between patients. Moreover, the compactness of the disposable devices allows them to be attached on the ETT circuit and not occupy bedside space and an electric power cable connection.

Laryngeal mask airway devices are useful in facilitating lung ventilation by forming a low-pressure seal around the patient's laryngeal inlet, thereby avoiding the known harmful effects of ETT devices, which form a seal within the trachea. Laryngeal mask airway devices have become standard medical devices, instead of ETT devices, for rapidly and reliably establishing an unobstructed airway in a patient in emergency situations and in the administration of anesthetic gases.

During general anesthesia, pulmonary ventilation is secured with an ETT device or by a laryngeal mask airway device, and attention to the risk of complications related to a high intracuff pressure is important. When the cuff-to-tracheal wall pressure exceeds the tracheal capillary pressure (130-140 cm H2O) for approximately 15 minutes, the tracheal mucous membrane becomes ischemic. The intracuff pressure approximates the cuff-to-tracheal wall pressures in high volume/low pressure cuffs, and a cuff pressure below 120 cm H2O is recommended to prevent ischemic injury. In addition, recurrent laryngeal nerve palsy has been demonstrated in up to 5% of patients after intubation, and a high cuff pressure is suspected as contributing to this complication. Similarly, in patients provided with a laryngeal mask, a high cuff pressure may lead to palsy of the lingual, hypoglossal, and recurrent laryngeal nerves, and postoperative sore throat.

PCT Publication WO 2017/153988 to Zachar et et al. describes a cuff pressure stabilizer that includes an inflation lumen proximal port connector, which is shaped to form an air-tight seal with an inflation lumen proximal port of a catheter additionally having an inflatable cuff and an inflation lumen; a fluid reservoir; a liquid column container, which is (a) open to the atmosphere at at least one site along the liquid column container, (b) in fluid communication with the fluid reservoir, and (c) in communication with the inflation lumen proximal port connector via the fluid reservoir; and a liquid, which is contained (a) in the fluid reservoir, (b) in the liquid column container, or (c) partially in the fluid reservoir and partially in the liquid column container, and which has a density of between 1.5 and 5 g/cm3 at 4 degrees Celsius at 1 atm. WO2014/145411 describes a a system for inflating and monitoring pressure comprising a retaining cuff.

### SUMMARY OF THE APPLICATION

Applications of the present invention provide cuff pressure stabilizers for use with an airway ventilation device having an inflatable cuff. The same cuff pressure stabilizer, without requiring adjustment, calibration, or other configuration, is able to provide pressure stabilization to inflatable cuffs of both tracheal ventilation tubes and laryngeal mask airway devices, even though the cuffs of these devices are inflated to substantially different pressures. Typically, cuffs of tracheal ventilation tubes are inflated to 25-30 cm H2O, while cuffs of laryngeal mask airway devices are inflated to 40-60 cm H2O.

In some applications of the present invention, in order to provide this pressure stabilization over such a wide range of pressures, a cuff pressure stabilizer is provided that comprises an elastic balloon, which is in fluid communication with the inflatable cuff, and which is disposed inside a protective housing that is configured to provide a pressure-volume curve with certain characteristics.

During use of the cuff pressure stabilizers described above, a healthcare worker inflates the inflatable cuff of the airway ventilation device to an initial desired pressure. The cuff pressure stabilizer is configured to automatically mechanically and non-electrically stabilize the pressure in the inflatable cuff to within a clinically-acceptable range above and below the initial desired pressure, so long as the initial desired pressure is within the normal clinically-acceptable range for cuffs of tracheal ventilation tubes or laryngeal mask airway devices.

The present invention will be more fully understood from the following detailed description of embodiments thereof, taken together with the drawings, in which:

### BRIEF DESCRIPTION OF THE DRAWINGS

Figs. 1A-C are schematic illustrations of a cuff pressure stabilizer for use with an airway ventilation device, in accordance with respective applications of the present invention;
Figs. 2A-B are additional schematic illustrations of the cuff pressure stabilizer of Fig. 1A, in accordance with an application of the present invention;
Figs. 3A-D are schematic illustrations of the cuff pressure stabilizer of Fig. 1A with an inflatable portion of a balloon thereof inflated with different respective volumes, in accordance with an application of the present invention;
Fig. 4 includes a pressure-volume curve, in accordance with an application of the present invention;
Fig. 5 is a schematic illustration of another cuff pressure stabilizer for use with an airway ventilation device, in accordance with an application of the present invention;
Fig. 6 is a cross-sectional view of the cuff pressure stabilizer of Fig. 5, in accordance with an application of the present invention;
Figs. 7A-C are schematic cross-sectional illustrations of the cuff pressure stabilizer of Fig. 5 with two elastic balloons thereof inflated at different pressures, in accordance with an application of the present invention;
Fig. 8A includes "confined" and "free" pressure-volume curves of a first inflatable portion of a first elastic balloon of the cuff pressure stabilizer of Fig. 5, in accordance with an application of the present invention;
Fig. 8B includes "confined" and "free" pressure-volume curves of a second inflatable portion of a second elastic balloon of the cuff pressure stabilizer of Fig. 5, in accordance with an application of the present invention;
Fig. 9 includes an aggregate pressure-volume curve of the cuff pressure stabilizer of Fig. 5, in accordance with an application of the present invention;
Fig. 10 is a schematic cross-sectional illustration of yet another cuff pressure stabilizer for use with an airway ventilation device, in accordance with respective applications of the present invention;
Fig. 11 is a schematic cross-sectional illustration of still another cuff pressure stabilizer for use with an airway ventilation device, in accordance with respective applications of the present invention;
Figs. 12A-C are schematic cross-sectional illustrations of another pressure stabilizer with an elastic balloon thereof inflated at different pressures, in accordance with an application of the present invention;
Figs. 13A-B are schematic illustrations of an alternative configuration of the cuff pressure stabilizer of Figs. 1A-3D, in accordance with an application of the present invention;
Fig. 13C is a schematic illustration of another alternative configuration of the cuff pressure stabilizer of Figs. 1A-3D, in accordance with an application of the present invention;
Fig. 14 is a schematic illustration of another cuff pressure stabilizer, in accordance with an application of the present invention;
Figs. 15A-B are cross-sectional views of Fig. 14 taken along lines XV-XV, in accordance with an application of the present invention;
Figs. 16A-C are schematic illustrations of the cuff pressure stabilizer of Figs. 14-15B with an inflatable portion of a balloon thereof inflated with different respective volumes, in accordance with an application of the present invention;
Figs. 17A-B are schematic illustrations of a protective housing of the cuff pressure stabilizer of Figs. 14-15B in locked and unlocked states, in accordance with an application of the present invention;
Fig. 18 includes three pressure-volume curves, in accordance with respective applications of the present invention; and
Figs. 19A-B are schematic illustrations of a pressure-sensing device for use with an airway ventilation device 10, in accordance with an application of the present invention.

### DETAILED DESCRIPTION OF APPLICATIONS

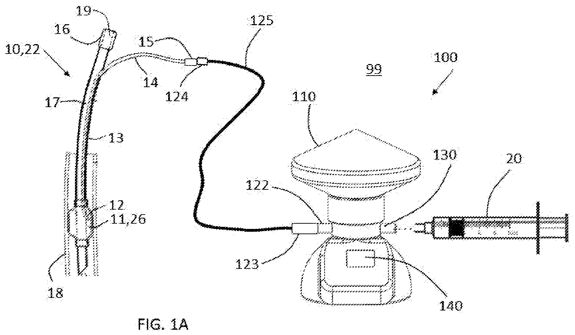

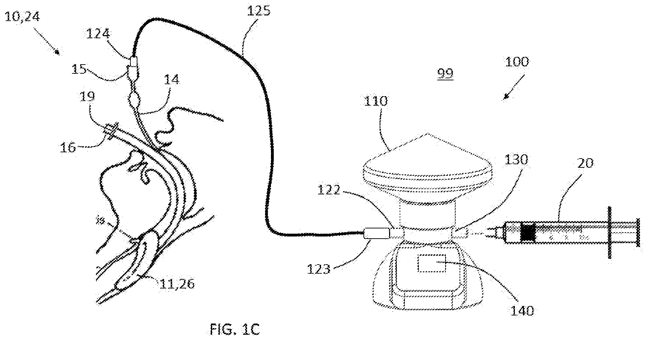

Figs. 1A-C are schematic illustrations of a cuff pressure stabilizer 100 for use with an airway ventilation device 10, in accordance with respective applications of the present invention. For example, airway ventilation device 10 may be a tracheal ventilation tube 22, such as shown in Figs. 1A-B, or a laryngeal mask airway device 24, such as shown in Fig. 1C. Cuff pressure stabilizer 100 is for use in contact with the atmosphere 99 (i.e., ambient air) of the Earth.

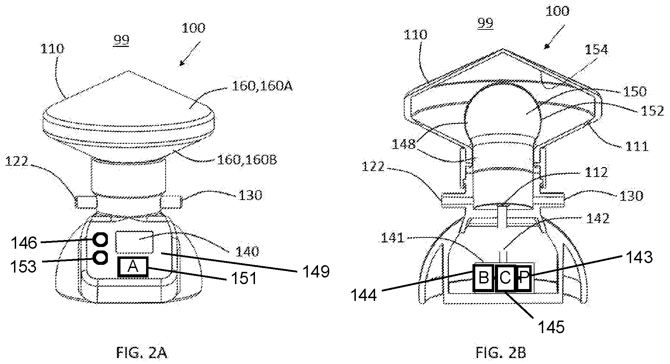

Figs. 1A-C also show (a) airway ventilation device 10, which is not a component of cuff pressure stabilizer 100, (b) an external inflation source 20, such as a syringe, which is typically not a component of cuff pressure stabilizer 100, and (c) one or more connector tubes, described hereinbelow, which are optionally a component of cuff pressure stabilizer 100 (and may be removably or permanently coupled to cuff pressure stabilizer 100). Cuff pressure stabilizer 100 typically comprises a stabilizer port 122, which is in fluid communication with elastic balloon 148, described hereinbelow with reference to Figs. 2A-B, and is configured to be coupled to the one or more connector tubes. The one or more connector tubes typically comprise a connector tube 125, which comprises an inflation lumen proximal port connector 124 that is shaped to form an air-tight seal with inflation lumen proximal port 15 of airway ventilation device 10, described immediately below. For some applications, inflation lumen proximal port connector 124 comprises a male conical fitting with a taper. For some applications, the taper is at least a 5% taper. For some applications, the taper is a 6% taper, and the male conical fitting with the 6% taper complies with International Standard ISO 594-1:1986, which is the standard for connections to conventional inflation lumen proximal ports of tracheal ventilation tubes and laryngeal mask airway masks.

Airway ventilation device 10 comprises an inflatable cuff 11, an inflation lumen 13, and an inflation lumen proximal port 15. Inflatable cuff 11 may comprise, for example, a balloon. Airway ventilation device 10 typically further comprises a cuff inflation lumen distal port 12, an airway ventilation tube ventilation port 16, an airway ventilation tube ventilation lumen 17, and an airway ventilation tube ventilator connection 19. For some applications, airway ventilation device 10 further comprises an inflating tube 14, which couples inflation lumen 13 in fluid communication with inflation lumen proximal port 15.

Reference is made to Figs. 1A-B. In these configurations, airway ventilation device 10 is a tracheal ventilation tube 22, and inflatable cuff 11 is an inflatable cuff 26 mounted on tracheal ventilation tube 22, typically near a distal end of the tracheal ventilation tube, e.g., within 3 cm, such as within 1 cm, of the distal end. In these configurations, inflatable cuff 26 typically comprises a nearly non-compliant material, and/or typically has a volume of between 5 and 20 cc, depending on the size of airway ventilation device 10. Tracheal ventilation tube 22 is schematically shown inserted into a trachea 18, and inflatable cuff 26 is inflatable into sealing contact with the inner surface of trachea 18. As used in the present application, including in the claims and the Inventive Concepts, a "tracheal ventilation tube" comprises an endotracheal tube (ETT) or a tracheostomy tube.

Reference is made to Fig. 1C. In this configuration, airway ventilation device 10 is a laryngeal mask airway device 24, and inflatable cuff 11 is an inflatable cuff 28 (which is typically annular) that is insertable through a mouth of a patient to an inserted location within the patient, such that an anterior side of the cuff forms a seal around a laryngeal inlet of the patient upon inflation of the cuff. When cuff 28 is inflated to a working medium pressure, the laryngeal mask airway device is suitable for facilitating lung ventilation. For example, the working medium pressure may be between 15 and 60 cm H2O, such as between 20 and 60 cm H2O, e.g., between 25 and 55 cm H2O, such as between 40 and 50 cm H2O. In this configuration, inflatable cuff 28 typically has a volume of between 25 and 50 cc, depending on the size of laryngeal mask airway device 24.

Reference is made to Figs. 1A and 1C. In these configurations, cuff pressure stabilizer 100 further comprises an inflation inlet port 130, which is in fluid communication with elastic balloon 148, described hereinbelow with reference to Figs. 2A-B. Inflation inlet port 130 is configured to be coupled in fluid communication with external inflation source 20. In these configurations, connector tube 125 typically further comprises a stabilizer-port connector 123, which is configured to be coupled in fluid communication with stabilizer port 122.

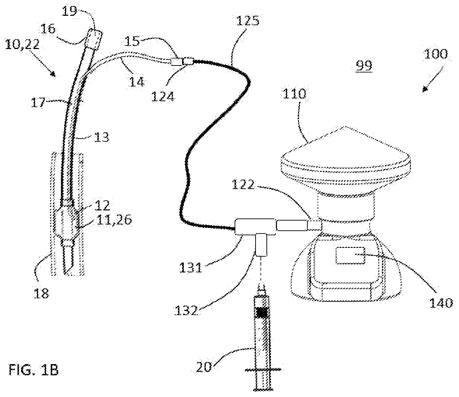

Reference is made to Fig. 1B. In this configuration, cuff pressure stabilizer 100 further comprises an inlet junction 131, which couples in fluid communication: connector tube 125, an inflation inlet port 132, first connector tube 133, and stabilizer port 122. Inflation inlet port 132 is configured to be coupled in fluid communication with external inflation source 20. In this configuration, cuff pressure stabilizer 100 typically does not comprise inflation inlet port 130, described hereinabove with reference to Figs. 1A and 1C. Alternatively, inlet junction 131 is provided, but is not a component of cuff pressure stabilizer 100. In an alternative configuration (not shown), the configuration described with reference to Fig. 1B is combined with laryngeal mask airway device 24, described with reference to in Fig. 1C, *mutatis mutandis.*

Reference is still made to Fig. 1A-C, and is additionally made to Figs. 2A-B, which are additional schematic illustrations of cuff pressure stabilizer 100, in accordance with an application of the present invention. Fig. 2B is a cross-section of Fig. 2A. Figs. 2A-B show the configuration of cuff pressure stabilizer 100 shown in Fig. 1A.

Cuff pressure stabilizer 100 comprises:
- stabilizer port 122, described hereinabove, which is configured to be coupled in fluid communication with inflation lumen proximal port 15 of airway ventilation device 10;
- a protective housing 110; and
- an elastic balloon 148, which is in fluid communication with stabilizer port 122, and which is arranged such that an inflatable portion 150 of balloon 148 is disposed inside protective housing 110 (balloon 148 may include other portions, such as the neck thereof, that are not inflatable because they are constrained from inflating, e.g., by the casing of cuff pressure stabilizer 100).

Protective housing 110 is typically more rigid than elastic balloon 148. For example, protective housing 110 may have a durometer hardness that is at least 3 times (e.g., at least 5 times) greater than a durometer hardness of elastic balloon 148. For example, the durometer hardness may be measured in Shore, such as Shore A, or another scale.

For some applications, protective housing 110 is substantially rigid. As used in the present application, including in the claims and the Inventive Concepts, "substantially rigid," when referring to protective housing 110, means that the protective housing, when disposed in atmosphere 99, does not materially deform at least when the pressure in balloon 148 is between 0 and 120 cm H2O. For some applications, the volume of the protective housing does not change by more than 1% when the pressure in the balloon increases from 0 cm H2O to 120 cm H2O.

For some applications, protective housing 110 is opaque.

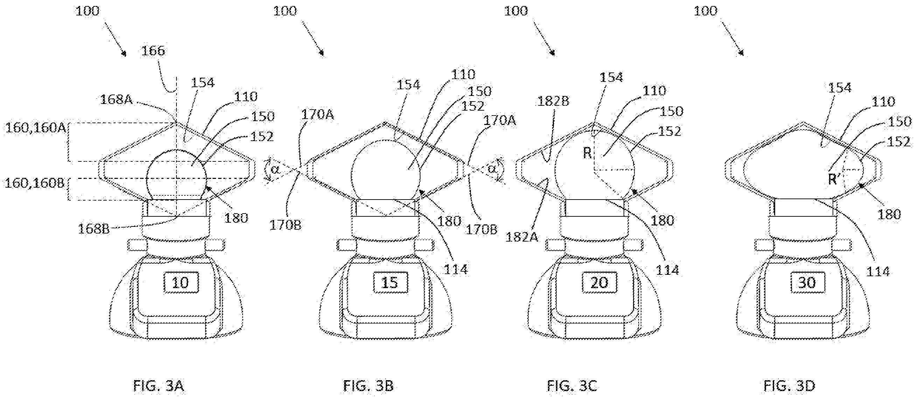

Inflatable portion 150 of balloon 148 is shaped so as to define an inflation inlet 114 that is in fluid communication with stabilizer port 122, such as shown in Figs. 3B-D, and a proximal surface of protective housing 110 is typically shaped so as to define an inflation opening 112 aligned with inflation inlet 114, such that inflatable portion 150 of balloon 148 is inflatable via inflation opening 112 of protective housing 110.

Reference is still made to Figs. 1A-C and 2A-B, and is additionally made to Figs. 3A-D, which are schematic illustrations of cuff pressure stabilizer 100 with inflatable portion 150 of balloon 148 inflated with different respective volumes, in accordance with an application of the present invention. Figs. 3A-D show the configuration of cuff pressure stabilizer 100 shown in Fig. 1A. For some applications, protective housing 110 is shaped and inflatable portion 150 of balloon 148 is configured such that:
- when inflatable portion 150 of balloon 148 contains a base low-pressure volume V_{B} of air, (a) inflatable portion 150 of balloon 148 has a base low pressure of 10 cm H2O, and (b) none of or less than 10% of an outer surface 152 of inflatable portion 150 of balloon 148 touches (i.e., comes in direct physical contact with) an inner surface 154 of protective housing 110, such as schematically illustrated in Fig. 3A,
- when inflatable portion 150 of balloon 148 contains a first-medium-pressure volume V₁ of air, (a) inflatable portion 150 of balloon 148 has a first-medium pressure of 15 cm H2O, and (b) none of or less than 15% of outer surface 152 of inflatable portion 150 of balloon 148 touches (i.e., comes in direct physical contact with) inner surface 154 of protective housing 110, such as schematically illustrated in Fig. 3B; the first-medium-pressure volume V₁ of air equals the sum of (a) the base low-pressure volume V_{B} of air and (b) a first incremental quantity Q₁ of air of less than 10 cc, and
- when inflatable portion 150 of balloon 148 contains a second-medium-pressure volume V₂ of air, (a) inflatable portion 150 of balloon 148 has a second-medium pressure of 30 cm H2O, and (b) at least 20% of outer surface 152 of inflatable portion 150 of balloon 148 touches a portion of inner surface 154 of protective housing 110, such as schematically illustrated in Fig. 3D; the second-medium-pressure volume V₂ of air equals the sum of (a) the base low-pressure volume V_{B} of air and (b) a second incremental quantity Q₂ of air that is between 10 cc and 50 cc, e.g., between 10 and 40 cc, such as between 10 and 30 cc.

For some applications, when inflatable portion 150 of balloon 148 contains the second-medium-pressure volume V₂ of air, no more than 50% of outer surface 152 of inflatable portion 150 of balloon 148 touches a portion of inner surface 154 of the protective housing 110.

Fig. 3C schematically illustrates inflatable portion 150 of balloon 148 containing another medium-pressure volume of air (greater than first-medium-pressure volume V₁ and less than second-medium-pressure volume V₂), such that inflatable portion 150 of balloon 148 has a medium pressure of 20 cm H2O, and less than 15% of outer surface 152 of inflatable portion 150 of balloon 148 touches inner surface 154 of protective housing 110.

For example, the above-mentioned base low-pressure volume V_{B} of air may be at least 2 cc, no more than 6 cc, and/or between 2 and 6 cc. For example, the above-mentioned first incremental quantity Q₁ of air may be at least 2 cc, no more than 10 cc (e.g., no more than 7 cc), and/or between 2 and 10 cc, such as between 2 and 7 cc. For example, the above-mentioned second-medium incremental quantity Q₂ of air may be at least 10 cc (e.g., at least 20 cc), no more than 50 cc (e.g., no more than 40 cc), and/or between 10 and 50 cc, such as between 20 and 40 cc.

Alternatively or additionally, for some applications, protective housing 110 is shaped and inflatable portion 150 of balloon 148 is configured such that:
- when inflatable portion 150 of balloon 148 contains a base low-pressure volume V_{B} of air, inflatable portion 150 of balloon 148 has a base low pressure of 10 cm H2O, such as schematically illustrated in Fig. 3A,
- when inflatable portion 150 of balloon 148 contains a first-medium-pressure volume V₁ of air, (a) inflatable portion 150 of balloon 148 has a first-medium pressure of 15 cm H2O, and (b) none of or less than 15% of outer surface 152 of inflatable portion 150 of balloon 148 touches (i.e., comes in direct physical contact with) inner surface 154 of protective housing 110, such as schematically illustrated in Fig. 3B; the first-medium-pressure volume V₁ of air equals the sum of (a) the base low-pressure volume V_{B} of air and (b) a first incremental quantity of air, typically less than 10 cc, and
- when inflatable portion 150 of balloon 148 contains a second-medium-pressure volume V₂ of air, (a) inflatable portion 150 of balloon 148 has a second-medium pressure, and (b) at least 20% of outer surface 152 of inflatable portion 150 of balloon 148 touches a portion of inner surface 154 of protective housing 110, such as schematically illustrated in Fig. 3D; the second-medium-pressure volume V₂ of air equals the sum of (a) the base low-pressure volume V_{B} of air and (b) a second incremental quantity of air that is between 1.1 and 3 times the first incremental quantity of air.

Protective housing 110 is shaped so as to define at least one opening 111 therethrough to the atmosphere 99 (labeled in Fig. 2B), in order to maintain air pressure within protective housing 110 but outside balloon 148 at approximately atmospheric pressure. For some applications, protective housing 110 has a volume of at least 20 cc (e.g., at least 30 cc), no more than 80 cc (e.g., no more than 60 cc), and/or between 20 and 80 cc, such as between 30 and 60 cc.

Reference is again made to Figs. 2A-B and 3A-D. For some applications, inner surface 154 of protective housing 110 is shaped so as to include a frustoconical portion 160 (labeled in Figs. 2A and 3A). Balloon 148 is arranged such that inflatable portion 150 of balloon 148 is disposed inside protective housing 110, typically such that:
- none or less than 15% of outer surface 152 of inflatable portion 150 of balloon 148 touches frustoconical portion 160 when inflatable portion 150 of balloon 148 is inflated to a first-medium pressure of 15 cm H2O, such as schematically illustrated in Fig. 3B, and
- at least 20% of outer surface 152 of inflatable portion 150 of balloon 148 touches at least a portion of frustoconical portion 160 when inflatable portion 150 of balloon 148 is inflated to a second-medium pressure greater than the first-medium pressure, such as schematically illustrated in Fig. 3D.

For some applications, frustoconical portion 160 of inner surface 154 of protective housing 110 that comes into contact with balloon 148 when balloon 148 is inflated to a medium pressure of 50 cm H2O has an area of at least 10 cm2, no more than 60 cm2, and/or between 10 and 60 cm2. For some applications, protective housing 110 is cylindrically symmetric about a central longitudinal axis 166 defined by frustoconical portion 160.

For some applications, frustoconical portion 160 is a first frustoconical portion 160A, and protective housing 110 is shaped such that inner surface 154 includes a second frustoconical portion 160B. First and second frustoconical portions 160A and 160B geometrically define different respective apices 168A and 168B. (It is to be understood that for a frustoconical portion that is not conical, the apex is the geometric apex of the portion of the cone cut off to produce the frustum that defines the frustoconical portion.) Optionally, first and second frustoconical portions 160A and 160B share a common central longitudinal axis 166, such as shown. Alternatively, first and second frustoconical portions 160A and 160B do not share a common central longitudinal axis (configuration not shown).

First and second frustoconical portions 160A and 160B geometrically define respective cones 170A and 170B (portions of which are labeled in Fig. 3B). For some applications, cones 170A and 170B intersect each other at one or more angles α (alpha), at least one of which is greater than 45 degrees. Typically, at least one of the angles is less than 90 degrees. For some applications, all of the angles are greater than 45 degrees, and/or all of the angles are less than 90 degrees. For applications in which first and second frustoconical portions 160A and 160B share common central longitudinal axis 166, such as shown, the respective cones 170A and 170B geometrically defined by first and second frustoconical portions 160A and 160B intersect each other at exactly one angle α (alpha). As used in the present application, including in the claims and the Inventive Concepts, "geometrically defined" means that the shape is defined abstractly in geometry, but not necessarily as a structural element of the device; for example, cones 170A and 170B are not necessarily structural elements of protective housing 110, although they could be. As used in the present application, including in the claims and the Inventive Concepts, the angle between two geometrical shapes is the smaller of the two supplementary angles between the two geometrical shapes, or equals 90 degrees if the two geometrical shapes are perpendicular.

For some applications, balloon 148 is arranged such that inflatable portion 150 of balloon 148 is disposed inside protective housing 110 such that as inflatable portion 150 of balloon 148 is inflated from the first-medium pressure toward the second-medium pressure, outer surface 152 of inflatable portion 150 of balloon 148 increases contact with second frustoconical portion 160B before increasing contact with first frustoconical portion 160A.

For some applications, protective housing 110 is shaped such that frustoconical portion 160 is part of a conical portion of inner surface 154. For example, first frustoconical portion 160A is illustrated as part of a conical portion of inner surface 154.

For some applications, inner surface 154 of protective housing 110 includes a proximal portion 182A that faces generally distally, and a distal portion 182B that faces generally proximally toward proximal portion 182A (labeled in Fig. 3C). One of proximal and distal portions 182A and 182B of inner surface 154 includes frustoconical portion 160. For some applications, a cone geometrically defined by frustoconical portion 160 intersects the other one of proximal and distal portions 182A and 182B of inner surface 154 at one or more angles, at least one of which is greater than 45 degrees.

For some applications, the other one of proximal and distal portions 182A and 182B of inner surface 154 is generally flat (configuration not shown). Frustoconical portion 160 geometrically defines a cone, which, for some of these applications, intersects the other one of proximal and distal portions 182A and 182B of inner surface 154 at one or more angles, e.g., at exactly one angle. At least one (e.g., all) of the one or more angles is greater than 45 degrees.

For some applications, frustoconical portion 160 is a first frustoconical portion 160A, and the other one of proximal and distal portions 182A and 182B of inner surface 154 defines a second frustoconical portion 160B. For some of these applications, respective cones geometrically defined by first and second frustoconical portions 160A and 160B intersect each other at one or more angles, e.g., at exactly one angle. At least one (e.g., all) of the one or more angles is greater than 45 degrees.

Reference is made to Figs. 1A-C, 2A-B, and 3A-D. For some applications, cuff pressure stabilizer 100 further comprises an electronic pressure measurement circuit 141 (labeled in Fig. 2B), comprising a pressure sensor 143 (labeled in Fig. 2B), which is configured to sense a pressure in inflatable portion 150 of balloon 148 (e.g., via an air inlet 142 for fluid communication between the pressure sensor and the balloon). The pressure sensor is disposed in balloon 148 or in a volume that is in fluid communication with balloon 148. Cuff pressure stabilizer 100 further comprises a pressure display 140, which is configured to display the pressure sensed by the pressure sensor, and is typically integrated with an external casing 149 of cuff pressure stabilizer 100, such as shown in Fig. 2A. Pressure display 140 may be digital or analog. It is noted that pressure sensor 143 and pressure display 140 only sense and display the pressure, respectively, but are not involved in setting or otherwise regulating the pressure in balloon 148 or inflatable cuff 11; in other words, the cuff pressure stabilizer 100 automatically mechanically and non-electrically stabilizes the pressure in inflatable cuff 11 without input from pressure sensor 143.

Electronic pressure measurement circuit 141 and display 140 comprise: pressure sensor 143 (labeled in Fig. 2B), a battery power supply 144 (labeled in Fig. 2B), an electronic controller 145 (labeled in Fig. 2B), a turn-ON switch 146 (labeled in Fig. 2A), and display 140 (labeled in Fig. 2A). For some applications, electronic pressure measurement circuit 141 takes a pressure measurement at time intervals greater than 10 seconds and less than 5 minutes (such as once per 30 seconds, or once per 60 seconds). For some applications, battery power supply 144 drains within less than 30 days of use (such as less than 14 days, or less than 7 days). This feature ensures the disposability of the device within the intended time limit of single-patient residence in hospital intensive care. For some applications, turn-ON switch 146 cannot be turned off to stop the battery drain after initial turn-ON.

For some applications, cuff pressure stabilizer 100 further comprises an alarm output 151 (shown in Fig. 2A), which is configured to generate a visual and/or audible signal (e.g., comprising a light source, such as an LED, for generating the visual signal, and/or a sound generator for generating the audible signal). For example, alarm output 151 may comprise a light source that is configured to emit green light when the pressure sensed by pressure sensor 143 is within a deviation value of a pressure threshold, and to emit a red light when the pressure sensed by pressure sensor 143 exceeds the pressure threshold by at least the deviation value. For some of these applications, cuff pressure stabilizer 100 further comprises a user input interface 153, and electronic pressure measurement circuit 141 is configured to set a pressure threshold responsively to an input received from user input interface 153 (for example, the input may be activation, e.g., by depressing, of user input interface 153, when the current pressure is at a certain value, and the pressure threshold may be set to the current pressure value), and activate alarm output 151 whenever the pressure sensed by pressure sensor 143 exceeds the pressure threshold by at least a deviation value. Typically, the deviation value equals at least 2 cm H2O, such as at least 4 cm H2O, e.g., 5 cm H2O. The deviation value is typically not adjustable by the user, and may be preset as an absolute value, or calculated by electronic pressure measurement circuit 141, for example as a percentage of the pressure threshold.

For some applications electronic pressure measurement circuit 141 is configured, upon receiving a set-pressure input from user input interface 153, to set the pressure threshold equal to a current pressure sensed by pressure sensor 143 at a time of receipt of the set-pressure input. For example, user input interface 153 may comprise a single button, which generates the set-pressure input upon being depressed by the user. The user may inflate inflatable cuff 11 of airway ventilation device 10 to a desired pressure and then actuate user input interface 153 to generate the set-pressure input (e.g., by depressing the button). Alternatively, user input interface 153 may comprise one or more buttons (e.g., two buttons) that allow the user to increase or decrease the desired pressure threshold.

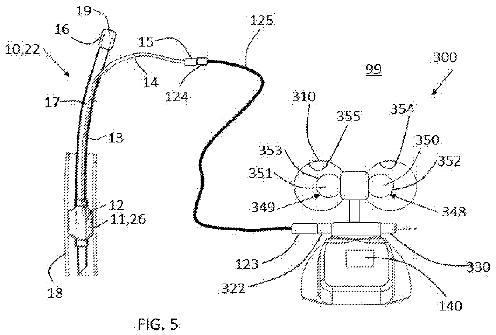

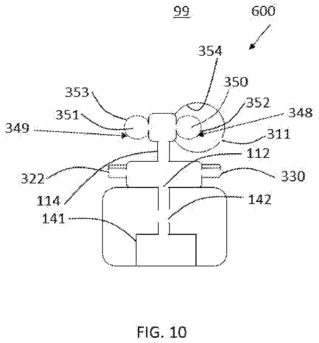

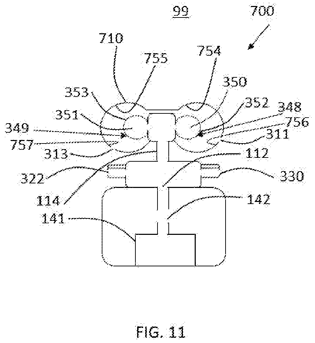

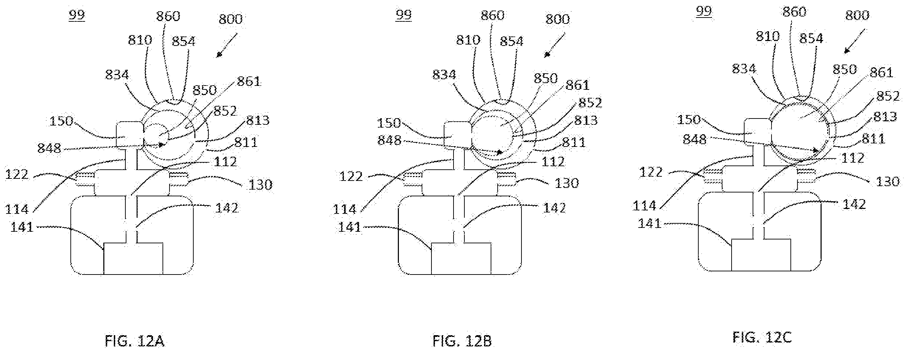

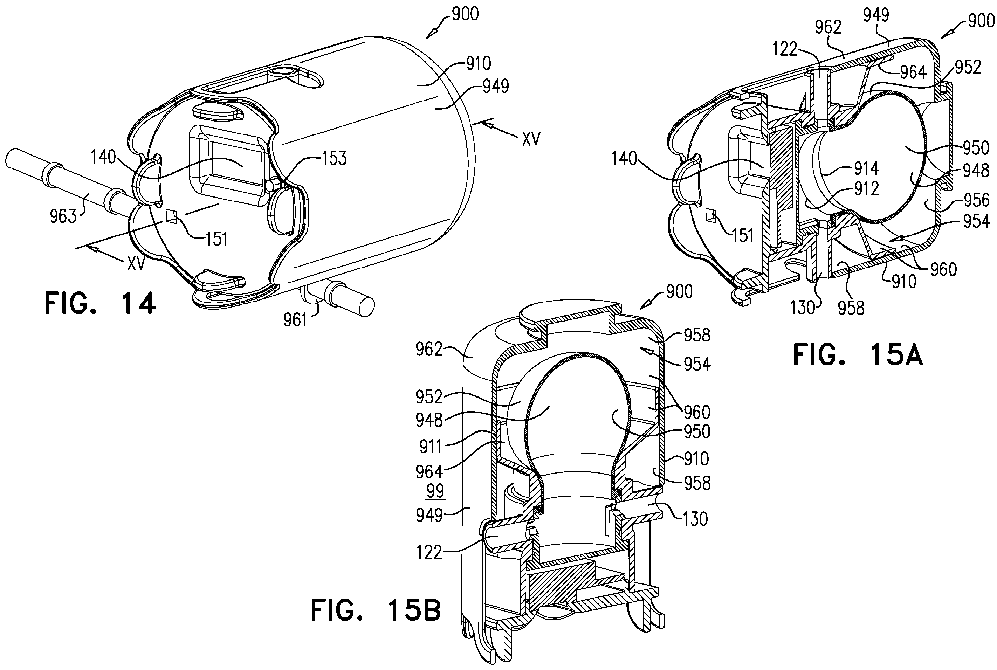

The pressure-sensing techniques described above with reference to Figs. 1A-C, 2A-B, and 3A-D may optionally be implemented by cuff pressure stabilizer 300, described hereinbelow with reference to Figs. 5-9; cuff pressure stabilizer 600, described hereinbelow with reference to Figs. 10; or cuff pressure stabilizer 700, described hereinbelow with reference to Fig. 11, *mutatis mutandis* (e.g., pressure sensor 143 is configured to sense a pressure in first and second inflatable portions 350 and 351 of first and second elastic balloons 348 and 349, respectively, of cuff pressure stabilizer 300, 600, or 700). In addition, the pressure-sensing techniques described above with reference to Figs. 1A-C, 2A-B, and 3A-D may optionally be implemented by cuff pressure stabilizer 800, described hereinbelow with reference to Figs. 12A-C, *mutatis mutandis* (e.g., pressure sensor 143 is configured to sense a pressure in inflatable portion 850 of elastic balloon 848 of cuff pressure stabilizer 800). In addition, the pressure-sensing techniques described above with reference to Figs. 1A-C, 2A-B, and 3A-D may optionally be implemented by cuff pressure stabilizer 900, described hereinbelow with reference to Figs. 14-17B, *mutatis mutandis.*

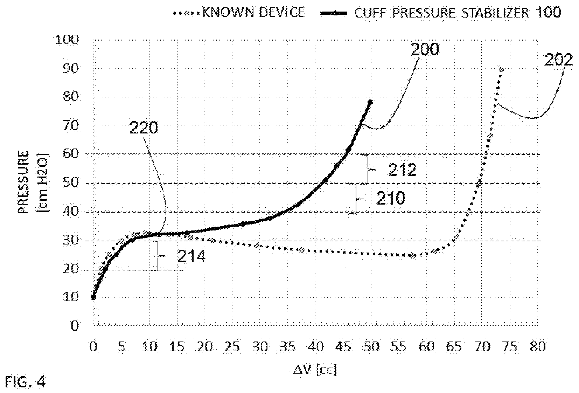

Reference is now made to Fig. 4, which includes a pressure-volume curve 200, in accordance with an application of the present invention. Fig. 4 also includes a known pressure-volume curve 202, measured in an experiment conducted on behalf of the inventors using the TRACOE^{®} smart Cuff Manager (TRACOE medical GmbH, Nieder-Olm, Germany), which was similar to the pressure-equalizing device described in the above-mentioned US Patent Application Publication 2015/0283343 to Schnell et al. Inflatable portion 150 of balloon 148 of cuff pressure stabilizer 100 is characterized by pressure-volume curve 200, which represents the pressure in inflatable portion 150 of balloon 148 when inflated with different incremental volumes of air (ΔV) beyond the base low-pressure volume V_{B} of air corresponding to the base low pressure of 10 cm H2O described hereinabove with reference to Figs. 3A-B. Pressure-volume curve 200 illustrated in Fig. 4 is an exemplary pressure-volume curve; a large number of additional pressure-volume curves having the general properties of pressure-volume curve 200 are possible, and are within the scope of the present invention.

For some applications, such as shown in Fig. 4, pressure-volume curve 200 does not include a local maximum pressure at any pressure between 20 and 50 cm H2O. By contrast, known pressure-volume curve 202 includes a local maximum pressure at about 32 cm H2O (at about 10 cc of incremental air). Alternatively, for other applications (not shown), pressure-volume curve 200 includes a local maximum pressure and a local minimum pressure at a greater incremental volume than the local maximum pressure, and (a) a pressure difference between the local maximum pressure and the local minimum pressure is less than 3 cm H2O, e.g., less than 2 cm H2O, and/or (b) a volume difference between the local maximum pressure and the local minimum pressure is less than 40 cc, e.g., less than 30 cc.

For some applications, an average rate of change of pressure-volume curve 200 over a first pressure interval 210 between 40 and 50 cm H2O is between 0.5 and 3 cm H2O / cc, such as between 0.5 and 2 cm H2O / cc, e.g., between 0.5 and 1 cm H2O / cc. By contrast, an average rate of change of known pressure-volume curve 202 over first pressure interval 210 is about 4 cm H2O / cc. Alternatively or additionally, for some applications, an average rate of change of pressure-volume curve 200 over a second pressure interval 212 between 50 and 60 cm H2O is between 0.5 and 3 cm H2O / cc, such as between 0.5 and 2 cm H2O / cc, e.g., between 0.5 and 1 cm H2O / cc. By contrast, an average rate of change of known pressure-volume curve 202 over second pressure interval 212 is about 6 cm H2O / cc. As is known in the mathematical arts, the "average rate of change" is the slope of the secant line joining respective points on the curve at the endpoints of the relevant interval.

Providing these relatively low average rates of change has the effect of stabilizing the pressure in inflatable cuff 28 of laryngeal mask airway device 24. Relatively small increases or decreases in the volume of inflatable cuff 28, for example caused by movement of cuff 28 against the patient's laryngeal inlet, result in corresponding decreases or increases in the volume of inflatable portion 150 of balloon 148. In the relevant typically desired pressure range of laryngeal mask airway cuffs of between 40 and 60 cm H2O, these changes in the volume of inflatable portion 150 have only minimal effect on the pressure in inflatable portion 150, and thus in inflatable cuff 28, because of the elasticity of balloon 148.

Alternatively or additionally, for some applications, an average rate of change of pressure-volume curve 200 over a pressure interval 214 between 20 and 30 cm H2O is between 0.3 and 5 cm H2O / cc, such as between 1 and 4 cm H2O / cc, e.g., between 1 and 3 cm H2O / cc. Providing these relatively low average rates of change has the effect of stabilizing the pressure in inflatable cuff 26 of tracheal ventilation tube 22. Relatively small increases or decreases in the volume of inflatable cuff 26, for example caused by movement of cuff 26 in trachea 18, result in corresponding decreases or increases in the volume of inflatable portion 150 of balloon 148. In the relevant typically desired pressure range of tracheal ventilation tube cuffs of between 20 and 30 cm H2O, these changes in the volume of inflatable portion 150 have only minimal effect on the pressure in inflatable portion 150, and thus in inflatable cuff 26, because of the elasticity of balloon 148.

Further alternatively or additionally, for some applications, pressure-volume curve 200 includes a rising point of inflection 220 at an inflection-point pressure of between 15 and 40 cm H2O, such as between 15 and 35 cm H2O or between 25 and 40 cm H2O, and/or at an incremental volume between 5 and 60 cc, such as between 10 and 30 cc. For these applications, pressure-volume curve 200 typically does not include a local maximum pressure at any pressure between 20 and 50 cm H2O. By contrast, known pressure-volume curve 202 does not include a rising point of inflection, and does include local maximum and minimum pressures. As is known in the mathematical arts, a "rising point of inflection" is a point of inflection at which the third derivative is positive, i.e., the curve is upwardflowing about the point and the curve changes from concave to convex from lower to higher pressures across the inflection point. As used in the present application, including in the claims and the Inventive Concepts, when referring to a pressure-volume curve, "concave" means concave downward, and "convex" means concave upward, in accordance with the common definitions in calculus.

For some applications, protective housing 110 is shaped and inflatable portion 150 of balloon 148 is configured such that:
- when inflatable portion 150 of balloon 148 is inflated at the base low pressure of 10 cm H2O, at least a base-low-pressure portion 180 of outer surface 152 of inflatable portion 150 of balloon 148 does not touch inner surface 154 of protective housing 110; base-low-pressure portion 180 excludes all portions of outer surface 152 of inflatable portion 150 within 5 mm of inflation inlet 114 of inflatable portion 150 of balloon 148 when inflatable portion 150 has the base low pressure of 10 cm H2O,
- when inflatable portion 150 of balloon 148 is inflated at all pressures greater than 10 cm H2O and less than the inflection-point pressure, none of base-low-pressure portion 180 of outer surface 152 of the inflatable portion of the balloon touches inner surface 154 of protective housing 110, and
- when inflatable portion 150 of balloon 148 is inflated at all pressures at and greater than the inflection-point pressure, base-low-pressure portion 180 of outer surface 152 of inflatable portion 150 of balloon 148 at least partially touches inner surface 154 of protective housing 110.

When inflatable portion 150 of balloon 148 contains the base low-pressure volume V_{B} of air, at least base-low-pressure portion 180 of outer surface 152 of inflatable portion 150 of balloon 148 does not touch inner surface 154 of protective housing 110. At all pressures greater than 10 cm H2O and less than a touching-point pressure, none of base-low-pressure portion 180 of outer surface 152 of inflatable portion 150 of balloon 148 touches inner surface 154 of protective housing 110. Typically, the touching-point pressure is less than 60 cm H2O; for example, the touching-point pressure may be greater than 15 cm H2O and less than 40 cm H2O, such as less than 30 cm H2O, or less than 25 cm H2O. For some applications, the touching-point pressure corresponds to the inflection-point pressure described above. At all pressures at and greater than the touching-point pressure, base-low-pressure portion 180 of outer surface 152 of inflatable portion 150 of balloon 148 at least partially touches inner surface 154 of protective housing 110.

For some applications, when inflatable portion 150 of balloon 148 is inflated at at all pressures at and greater than the touching-point pressure and less than 60 cm H2O, pressure-volume curve 200 is convex.

For some applications:
- when inflatable portion 150 of balloon 148 is inflated at a pressure of 20 cm H2O, a first area of base-low-pressure portion 180 of outer surface 152 of inflatable portion 150 of balloon 148 touches inner surface 154 of protective housing 110,
- when inflatable portion 150 of balloon 148 is inflated at a pressure of 30 cm H2O, a second area of base-low-pressure portion 180 of outer surface 152 of inflatable portion 150 of balloon 148 touches inner surface 154 of protective housing 110, and
- the second area equals at least 3 times the first area.

For some applications, pressure-volume curve 200 does not include any plateaus (i.e., horizontal portions where the pressure-volume curve has a constant value).

Reference is now made to Fig. 5, which is a schematic illustration of a cuff pressure stabilizer 300 for use with airway ventilation device 10, in accordance with an application of the present invention. For example, airway ventilation device 10 may be tracheal ventilation tube 22, such as shown in Fig. 5, or laryngeal mask airway device 24, such as shown in Fig. 1C, described hereinabove for cuff pressure stabilizer 100. Cuff pressure stabilizer 300 is for use in contact with the atmosphere 99 (i.e., ambient air) of the Earth.

Fig. 5 also shows (a) airway ventilation device 10, which is not a component of cuff pressure stabilizer 300, (b) external inflation source 20, such as a syringe, which is typically not a component of cuff pressure stabilizer 300, and (c) one or more connector tubes, described hereinbelow, which are optionally a component of cuff pressure stabilizer 300 (and may be removably or permanently coupled to cuff pressure stabilizer 300). Airway ventilation device 10 is described hereinabove with reference to Figs. 1A-C. Cuff pressure stabilizer 300 typically comprises a stabilizer port 322, which is in fluid communication with first and second elastic balloons 348 and 349, described hereinbelow with reference to Figs. 5, 6, and 7A-C, and is configured to be coupled to the one or more connector tubes. The one or more connector tubes typically comprise connector tube 125, which comprises inflation lumen proximal port connector 124 that is shaped to form an air-tight seal with inflation lumen proximal port 15 of airway ventilation device 10, described immediately below. Inflation lumen proximal port connector 124 is described hereinabove with reference to Figs. 1A-C.

For some applications, such as shown in Fig. 5, cuff pressure stabilizer 300 further comprises an inflation inlet port 330, which is in fluid communication with first and second elastic balloons 348 and 349, described hereinbelow with reference to Figs. 5-7C. Inflation inlet port 330 is configured to be coupled in fluid communication with external inflation source 20. In these configurations, connector tube 125 typically further comprises stabilizer-port connector 123, which is configured to be coupled in fluid communication with stabilizer port 322.

For other applications, such as shown in Fig. 1B for cuff pressure stabilizer 100, cuff pressure stabilizer 300 further comprises inlet junction 131, which couples in fluid communication: connector tube 125, an inflation inlet port 132, first connector tube 133, and stabilizer port 122. Inflation inlet port 132 is configured to be coupled in fluid communication with external inflation source 20. In this configuration, cuff pressure stabilizer 300 typically does not comprise inflation inlet port 330, described hereinabove with reference to Fig. 2. Alternatively, inlet junction 131 is provided, but is not a component of cuff pressure stabilizer 300. In an alternative configuration (not shown), the configuration described with reference to Fig. 1B is combined with laryngeal mask airway device 24, described with reference to in Fig. 1C, *mutatis mutandis.*

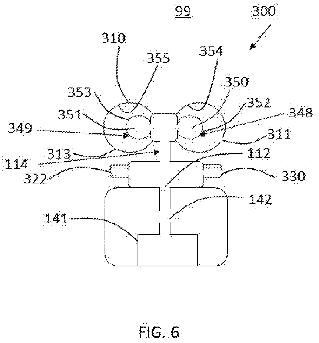

Reference is still made to Fig. 5, and is additionally made to Fig. 6, which is a cross-sectional view of cuff pressure stabilizer 300, in accordance with an application of the present invention. Cuff pressure stabilizer 300 comprises:
- stabilizer port 322, described hereinabove, which is configured to be coupled in fluid communication with inflation lumen proximal port 15 of airway ventilation device 10;
- housing 310, which comprises one or more internal protective surfaces 354 and 355; and
- first and second elastic balloons 348 and 349, which comprise respective first and second inflatable portions 350 and 351 that are in fluid communication with each other and with stabilizer port 322 (balloons 348 and 349 may include other portions, such as necks thereof, that are not inflatable because they are constrained from inflating, e.g., by housing 310 of cuff pressure stabilizer 300).

The one or more internal protective surfaces 354 and 355 are typically more rigid than first and second elastic balloons 348 and 349. For example, the one or more internal protective surfaces 354 and 355 may have a durometer hardness that is at least 3 times (e.g., at least 5 times) greater than a durometer hardness of first and second elastic balloons 348 and 349. For example, the durometer hardness may be measured in Shore, such as Shore A, or another scale.

For some applications, the one or more internal protective surfaces 354 and 355 are substantially rigid. As used in the present application, including in the claims and the Inventive Concepts, first and second inflatable portions 350 and 351 include the entireties of respective inflatable portions of first and second elastic balloons 348 and 349, and not arbitrary sub-portions thereof. As used in the present application, including in the claims and the Inventive Concepts, "substantially rigid," when referring to internal protective surfaces 354 and 355 of housing 310, means that the internal protective surfaces, when disposed in atmosphere 99, does no materially deform at least when the pressure in balloons 348 and 349 is between 0 and 120 cm H2O, i.e., the volume of housing 310 does not change by more than 1% when the pressure in balloon 348 and 349 increases from 0 cm H2O to 120 H2O.

For some applications, housing 310 is opaque.

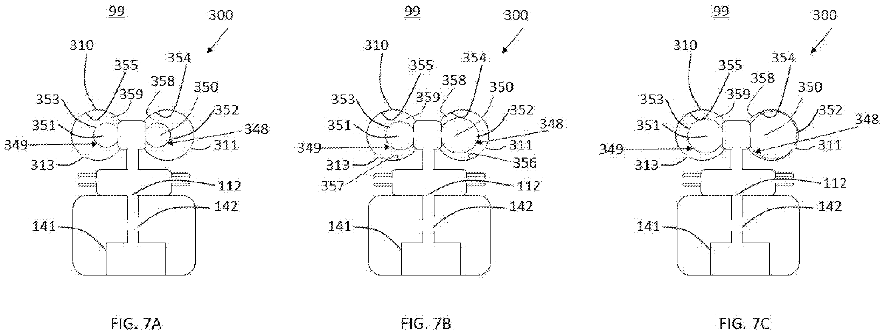

Reference is made to Figs. 7A-C, which are schematic cross-sectional illustrations of cuff pressure stabilizer 300 with balloons 348 and 349 inflated at different pressures, in accordance with an application of the present invention. Typically, the one or more internal protective surfaces 354 and 355 are shaped and first and second inflatable portions 350 and 351 are configured such that:
(a) when each of first and second inflatable portions 350 and 351 is inflated at a first-medium pressure of 15 cm H2O, such as, for example, could be the pressure illustrated in Fig. 7B:
   (i) none of or less than 15% of a first entire outer surface 352 of first inflatable portion 350 touches the one or more internal protective surfaces 354 and 355, i.e., first inflatable portion 350 is not meaningfully confined by the one or more internal protective surfaces 354 and 355, and
   (ii) none of or less than 15% of a second entire outer surface 353 of second inflatable portion 351 touches the one or more internal protective surfaces 354 and 355, i.e., second inflatable portion 351 is not meaningfully confined by the one or more internal protective surfaces 354 and 355, and
(b) when each of first and second inflatable portions 350 and 351 is inflated at a second-medium pressure of 30 cm H2O:
   (i) at least 20% of first entire outer surface 352 touches a first portion 356 (labeled in Fig. 7B) of the one or more internal protective surfaces 354 and 355, i.e., first inflatable portion 350 is at least somewhat confined by first portion 356 of the one or more internal protective surfaces 354 and 355, and
   (ii) none of or less than 15% of second entire outer surface 353 touches the one or more internal protective surfaces 354 and 355, i.e., second inflatable portion 351 is not meaningfully confined by the one or more internal protective surfaces 354 and 355.

For some applications, the one or more internal protective surfaces 354 and 355 are shaped and first and second inflatable portions 350 and 351 are configured such that when each of first and second inflatable portions 350 and 351 is inflated at a second-medium pressure of 50 cm H2O, such as, for example, could be the pressure illustrated in Fig. 7C:
(i) at least 50% (such as at least 75%) of first entire outer surface 352 touches first portion 356 of the one or more internal protective surfaces 354 and 355, i.e., first inflatable portion 350 is highly confined by first portion 356 of the one or more internal protective surfaces 354 and 355, and
(ii) none of or less than 20% of second entire outer surface 353 touches the one or more internal protective surfaces 354 and 355, i.e., second inflatable portion 351 is not meaningfully confined by the one or more internal protective surfaces 354 and 355.

For some applications, such as shown in Figs. 5-7C, first portion 356 of the one or more internal protective surfaces 354 and 355 surrounds at least 50% (such as least 75%, e.g., least 90%, such as at least 99%) of first entire outer surface 352 when first inflatable portion 350 is inflated at the first-medium pressure of 15 cm H2O. Alternatively, first portion 356 of the one or more internal protective surfaces 354 and 355 surrounds less than 50% of first entire outer surface 352 when first inflatable portion 350 is inflated at the first-medium pressure of 15 cm H2O (configuration not shown).

For some applications, such as shown in Figs. 5-7C, first portion 356 of the one or more internal protective surfaces 354 and 355 is generally spherical. Alternatively, first portion 356 has another shape, e.g., is generally ellipsoidal (configuration not shown).

For some applications, such as shown in Figs. 5-7C, a second portion 357 (labeled in Fig. 7B) of the one or more internal protective surfaces 354 and 355, which is entirely distinct from first portion 356 of the one or more internal protective surfaces 354 and 355, surrounds at least 50% (such as least 75%, e.g., least 90%, such as at least 99%) of second entire outer surface 353 when second inflatable portion 351 is inflated at the first-medium pressure of 15 cm H2O. Second portion 357 may protect second elastic balloon 349. Typically, second portion 357 does not meaningfully constrain or confine the expansion of second inflatable portion 351 of second elastic balloon 349 during normal use of cuff pressure stabilizer 300 at normal pressures. Alternatively, second portion 357 of the one or more internal protective surfaces 354 and 355, surrounds less than 50% of second entire outer surface 353 when second inflatable portion 351 is inflated at the first-medium pressure of 15 cm H2O.

For some applications, such as shown in Figs. 5-7C, second portion 357 of the one or more internal protective surfaces 354 and 355 is generally spherical. Alternatively, second portion 357 has another shape, e.g., is generally ellipsoidal (configuration not shown).

For some applications, such as shown in Figs. 5-7C, first portion 356 and second portion 357 of the one or more internal protective surfaces 354 and 355 are shaped so as to define first and second chambers 358 and 359 (labeled in Fig. 7A), respectively, and first and second chambers 358 and 359 are not in fluid communication with each other via housing 310. Alternatively, first and second inflatable portions 350 and 351 are disposed in a single chamber, such as described hereinbelow with reference to Fig. 11.

Typically, first portion 356 (e.g., first chamber 358) is shaped so as to define at least one opening 311 therethrough to the atmosphere 99, in order to maintain air pressure within first portion 356 (e.g., first chamber 358) but outside first elastic balloon 348 at approximately atmospheric pressure. Similarly, for configuration in which second portion 357 is provided, second portion 357 (e.g., second chamber 359) is shaped so as to define at least one opening 313 therethrough to the atmosphere 99, in order to maintain air pressure within second portion 357 (e.g., second chamber 359) but outside second elastic balloon 349 at approximately atmospheric pressure.

Typically, such as shown in Figs. 5-7C, the one or more internal protective surfaces 354 and 355 are shaped and first and second inflatable portions 350 and 351 are configured such that no portion of first entire outer surface 352 touches second entire outer surface 353 when each of first and second inflatable portions 350 and 351 is inflated at any pressure.

For some applications, the one or more internal protective surfaces 354 and 355 are shaped and first and second inflatable portions 350 and 351 are configured such that a second volume of second inflatable portion 351 equals between 80% and 120% (e.g., between 90% and 110%, such as between 95% and 105%, e.g., 100%) of a first volume of first inflatable portion 350 when each of first and second inflatable portions 350 and 351 is inflated at a base low pressure of 10 cm H2O, such as, for example, could be the pressure illustrated in Fig. 7A. For some of these applications, the one or more internal protective surfaces 354 and 355 are shaped and first and second inflatable portions 350 and 351 are configured such that the first volume equals at least 50% of the second volume when each of first and second inflatable portions 350 and 351 is inflated at the first-medium pressure of 15 cm H2O, such as, for example, could be the pressure illustrated in Fig. 7B.

For some of these applications, first and second inflatable portions 350 and 351 comprise respective different materials in order to provide the above-mentioned first and second volumes. Alternatively, (a) first and second inflatable portions 350 and 351 comprise a same material (i.e., the same exact material, not just the same type of material or class of materials), (b) first and second inflatable portions 350 and 351 have first and second average wall thicknesses, respectively, and (c) the second average wall thickness equals between 110% and 180% of the first average wall thickness.

For some applications, cuff pressure stabilizer 300 comprises electronic pressure measurement circuit 141, described hereinabove with reference to Figs. 1A-C, 2A-B, and 3A-D. The pressure sensor of electronic pressure measurement circuit 141 is configured to sense a pressure in first and second inflatable portions 350 and 351, and is disposed in first elastic balloon 348, second elastic balloon 349, or in a volume that is in fluid communication with first and second elastic balloons 348 and 349.

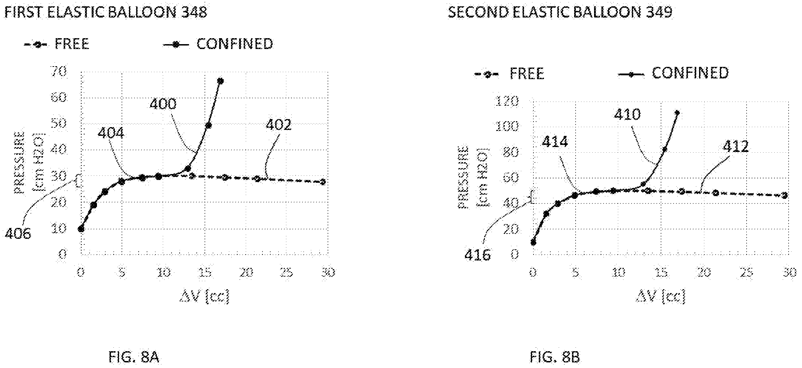

Reference is now made to Fig. 8A, which includes "confined" and "free" pressure-volume curves 400 and 402 of first inflatable portion 350 of first elastic balloon 348, in accordance with an application of the present invention. "Confined" and "free" pressure-volume curves 400 and 402 represent the pressure in first inflatable portion 350 of first elastic balloon 348 when inflated with different incremental volumes of air (ΔV) beyond a first base low-pressure volume V_{B1} of air corresponding to the base low pressure of 10 cm H2O described hereinabove with reference to Figs. 5-7C. Pressure-volume curves 400 and 402 illustrated in Fig. 8A are exemplary pressure-volume curves; a large number of additional pressure-volume curves having the general properties of pressure-volume curves 400 and 402 are possible, and are within the scope of the present invention.

First inflatable portion 350 of first elastic balloon 348 of cuff pressure stabilizer 300 is characterized by "confined" pressure-volume curve 400 when first inflatable portion 350 is disposed within first portion 356 of the one or more internal protective surfaces 354 and 355 and configured such that at least certain percentages of first entire outer surface 352 touches first portion 356 at certain pressures, as described hereinabove with reference to Figs. 7A-C.

By contrast, first inflatable portion 350 of first elastic balloon 348 of cuff pressure stabilizer 300 would be characterized by "free" pressure-volume curve 402 if first inflatable portion 350 were not disposed within first portion 356 or otherwise confined, i.e., unlike the configuration of cuff pressure stabilizer 300 described herein with reference to Figs. 5-7C. Thus, "free" pressure-volume curve 402 reflects physical, structural properties of first inflatable portion 350.

First inflatable portion 350 of first elastic balloon 348 of cuff pressure stabilizer 300 is characterized by a first pressure-volume curve 404 at all pressures up to 30 cm H2O whether or not first inflatable portion 350 is confined by first portion 356 of the one or more internal protective surfaces 354 and 355 at pressures greater than 30 cm H2O.

Reference is now made to Fig. 8B, which includes "confined" and "free" pressure-volume curves 410 and 412 of second inflatable portion 351 of second elastic balloon 349, in accordance with an application of the present invention. "Confined" and "free" pressure-volume curves 410 and 412 represent the pressure in second inflatable portion 351 of second elastic balloon 349 when inflated with different incremental volumes of air (ΔV) beyond a second base low-pressure volume V_{B2} of air corresponding to the base low pressure of 10 cm H2O described hereinabove with reference to Figs. 5-7C. Pressure-volume curves 410 and 412 illustrated in Fig. 8B are exemplary pressure-volume curves; a large number of additional pressure-volume curves having the general properties of pressure-volume curves 410 and 412 are possible, and are within the scope of the present invention.

Second inflatable portion 351 of second elastic balloon 349 of cuff pressure stabilizer 300 is characterized by "confined" pressure-volume curve 410 when second inflatable portion 351 is disposed within second portion 357 of the one or more internal protective surfaces 354 and 355 and configured such that at least certain percentages of second entire outer surface 353 touches second portion 357 at certain pressures. As mentioned above with reference to Figs. 7A-C, second portion 357 typically does not meaningfully constrain or confine the expansion of second inflatable portion 351 of second elastic balloon 349 during normal use of cuff pressure stabilizer 300 at normal pressures.

By contrast, second inflatable portion 351 of second elastic balloon 349 of cuff pressure stabilizer 300 would be characterized by "free" pressure-volume curve 412 if second inflatable portion 351 were not disposed within second portion 357 or otherwise confined, i.e., unlike the configuration of cuff pressure stabilizer 300 described herein with reference to Figs. 5-7C, but like the configuration of cuff pressure stabilizer 600, described hereinbelow with reference to Fig. 11. Thus, "free" pressure-volume curve 412 reflects physical, structural properties of second inflatable portion 351.

Second inflatable portion 351 of second elastic balloon 349 of cuff pressure stabilizer 300 is characterized by a first pressure-volume curve 414 at all pressures up to 50 cm H2O whether or not second inflatable portion 351 is confined by second portion 357 of the one or more internal protective surfaces 354 and 355 at pressures greater than 50 cm H2O.

Reference is made to both Figs. 8A and 8B. For some applications, the one or more internal protective surfaces 354 and 355 are shaped and first and second inflatable portions 350 and 351 are configured such that:
- (a) when first inflatable portion 350 contains first base low-pressure volume V_{B1} of air, first inflatable portion 350 has the base low pressure of 10 cm H2O, (b) first inflatable portion 350 is characterized by first pressure-volume curve 404 that represents the pressure in first inflatable portion 350 when inflated with different incremental volumes of air beyond the first base low-pressure volume V_{B1} of air, and (c) an average rate of change of the first pressure-volume curve over a first pressure interval 406 between 25 and 30 cm H2O is between 0.3 and 2 cm H2O / cc, and
- (a) when second inflatable portion 351 contains second base low-pressure volume V_{B2} of air, second inflatable portion 351 has the base low pressure of 10 cm H2O, (b) second inflatable portion 351 is characterized by second pressure-volume curve 414 that represents the pressure in second inflatable portion 351 when inflated with different incremental volumes of air beyond the second base low-pressure volume V_{B2} of air, and (c) an average rate of change of the second pressure-volume curve over a second pressure interval 416 between 40 and 50 cm H2O is between 0.2 and 2 cm H2O / cc.

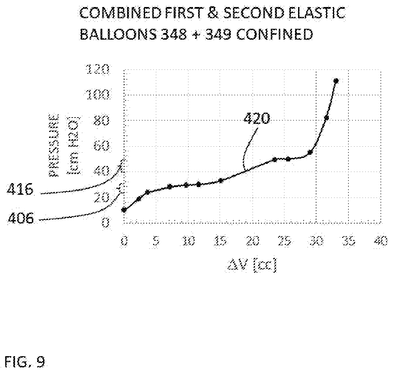

Reference is now made to Fig. 9, which includes an aggregate pressure-volume curve 420, in accordance with an application of the present invention. Cuff pressure stabilizer 300 is characterized by aggregate pressure-volume curve 420, which represents the pressure in first and second inflatable portions 350 and 351 when inflated, in aggregate, with different aggregate incremental volumes of air beyond a base aggregate low-pressure volume V_{BA} of air corresponding to the base low pressure of 10 cm H2O described hereinabove with reference to Figs. 5-7C. Aggregate pressure-volume curve 420 illustrated in Fig. 9 is an exemplary pressure-volume curve; a large number of additional pressure-volume curves having the general properties of aggregate pressure-volume curve 420 are possible, and are within the scope of the present invention.

For some applications, the one or more internal protective surfaces 354 and 355 are shaped and first and second inflatable portions 350 and 351 are configured such that:
- when first and second inflatable portions 350 and 351 contain, in aggregate, the base aggregate low-pressure volume V_{BA} of air, each of first and second inflatable portions 350 and 351 has the base low pressure of 10 cm H2O,
- cuff pressure stabilizer 300 is characterized by aggregate pressure-volume curve 420 that represents the pressure in first and second inflatable portions 350 and 351 when inflated, in aggregate, with different aggregate incremental volumes of air beyond the base aggregate low-pressure volume V_{BA} of air, and
- an average rate of change of the aggregate pressure-volume curve over first pressure interval 406 between 25 and 30 cm H2O is between 0.3 and 2 cm H2O / cc.

For some applications, the one or more internal protective surfaces 354 and 355 are shaped and first and second inflatable portions 350 and 351 are configured such that an average rate of change of the aggregate pressure-volume curve over second pressure interval 416 between 40 and 50 cm H2O is between 0.2 and 2 cm H2O / cc.

For some applications the one or more internal protective surfaces 354 and 355 are shaped and first and second inflatable portions 350 and 351 are configured such that aggregate pressure-volume curve 420 does not include a local maximum pressure at any pressure between 20 and 50 cm H2O, such as shown in Fig. 9. Alternatively, for some applications, the one or more internal protective surfaces 354 and 355 are shaped and first and second inflatable portions 350 and 351 are configured such that aggregate pressure-volume curve 420 includes a local maximum pressure and a local minimum pressure at a greater incremental volume than the local maximum pressure, and a pressure difference between the local maximum pressure and the local minimum pressure is less than 3 cm H2O (configuration not shown). Alternatively or additionally, for some applications, aggregate pressure-volume curve 420 includes a local maximum pressure and a local minimum pressure at a greater incremental volume than the local maximum pressure, and a volume difference between the local maximum pressure and the local minimum pressure is less than 40 cc (configuration not shown).

For some applications, the one or more internal protective surfaces 354 and 355 are shaped and first and second inflatable portions 350 and 351 are configured such that:
- each of first and second inflatable portions 350 and 351 has the base low pressure of 10 cm H2O when first and second inflatable portions 350 and 351 contain, in aggregate, the base aggregate low-pressure volume V_{BA} of air, and
- each of first and second inflatable portions 350 and 351 has the first-medium pressure of 15 cm H2O when first and second inflatable portions 350 and 351 contain, in aggregate, a first aggregate medium-pressure volume of air, and
- the first-medium-pressure volume of air equals the sum of (a) the base aggregate low-pressure volume V_{BA} of air and (b) a first aggregate incremental quantity of air of less than 10 cc.

For some applications the one or more internal protective surfaces 354 and 355 are shaped and first and second inflatable portions 350 and 351 are configured such that each of first and second inflatable portions 350 and 351 has the second-medium pressure of 30 cm H2O when first and second inflatable portions 350 and 351 contain, in aggregate, a second aggregate medium-pressure volume of air equal to the sum of (a) the base aggregate low-pressure volume V_{BA} of air and (b) a second aggregate incremental quantity of air that is between 10 cc and 60 cc, such as less than 30 cc, e.g., less than 15 cc.

In an application of the present invention, a cuff pressure stabilizer is provided that is identical to cuff pressure stabilizer 300 described hereinabove with reference to Figs. 5-9, except as follows. This cuff pressure stabilizer may implement any of the features of cuff pressure stabilizer 300 described hereinabove with reference to Figs. 5-9. This cuff pressure stabilizer comprises first and second elastic balloons, which comprise respective first and second inflatable portions that are in fluid communication with each other and with stabilizer port 322.

The one or more internal protective surfaces 354 and 355 of this cuff pressure stabilizer are shaped and the first and the second inflatable portions are configured such that a second volume of the second inflatable portion equals between 50% and 75% of a first volume of the first inflatable portion when each of the first and the second inflatable portions has a base low pressure of 10 cm H2O. For some applications, the first and the second inflatable portions comprise a same material, and the first and the second inflatable portions have first and second average wall thicknesses, respectively, that equal each other.

Reference is now made to Fig. 10, which is a schematic cross-sectional illustration of a cuff pressure stabilizer 600 for use with airway ventilation device 10, in accordance with respective applications of the present invention. Except as described hereinbelow, cuff pressure stabilizer 600 is identical to cuff pressure stabilizer 300 described hereinabove with reference to Figs. 5-9, and may implement any of the features thereof, and/or of the cuff pressure stabilizer described immediately hereinabove. Like reference numerals refer to like elements.

Cuff pressure stabilizer 600, unlike the configuration of cuff pressure stabilizer 300 illustrated in Figs. 5-7C, does not include second portion 357 of the one or more internal protective surfaces 354 and 355. Therefore, second entire outer surface 353 of second inflatable portion 351 is not surrounded by the one or more internal protective surfaces 354 and 355. This modification to cuff pressure stabilizer 300 typically does not affect the pressure-volume curves thereof because, as mentioned above with reference to Figs. 5-7C, second portion 357 of the one or more internal protective surfaces 354 and 355 typically in any event does not meaningfully constrain or confine the expansion of second inflatable portion 351 of second elastic balloon 349 during normal use of cuff pressure stabilizer 300 at normal pressures.

Reference is now made to Fig. 11, which is a schematic cross-sectional illustration of a cuff pressure stabilizer 700 for use with airway ventilation device 10, in accordance with respective applications of the present invention. Except as described hereinbelow, cuff pressure stabilizer 700 is identical to cuff pressure stabilizer 300 described hereinabove with reference to Figs. 5-9, and may implement any of the features thereof, and/or of the cuff pressure stabilizer described hereinabove immediately before the description of Fig. 10. Like reference numerals refer to like elements. Cuff pressure stabilizer 700 comprises a housing 710, which comprises one or more internal protective surfaces 754 and 755. Internal protective surfaces 754 and 755 are shaped so as to define first and second portions 756 and 757 that are similar to first and second portions 356 and 357 of cuff pressure stabilizer 300.

For some applications, housing 710 is opaque.

In cuff pressure stabilizer 700, unlike in the configuration of cuff pressure stabilizer 300 illustrated in Figs. 5-7C, first and second inflatable portions 350 and 351 are disposed in a single chamber 760 defined by internal protective surfaces 754 and 755. This modification to cuff pressure stabilizer 300 typically does not affect the pressure-volume curves thereof because the shape of first portion 756 of cuff pressure stabilizer 700 is similar to the shape of first portion 356 of cuff pressure stabilizer 300.

Reference is made to Figs. 12A-C, which are schematic cross-sectional illustrations of a cuff pressure stabilizer 800 with an elastic balloon 848 thereof inflated at different pressures, in accordance with an application of the present invention. Except as described hereinbelow, cuff pressure stabilizer 800 is similar cuff pressure stabilizer 300 described hereinabove with reference to Figs. 5-9, may implement any of the features thereof, and is for use with airway ventilation device 10 (e.g., tracheal ventilation tube 22 or laryngeal mask airway device 24). Like reference numerals refer to like elements.

Cuff pressure stabilizer 800 comprises:
- a stabilizer port 322, which is configured to be coupled in fluid communication with inflation lumen proximal port 15 of airway ventilation device 10;
- an expandable wall membrane 834, which is not in fluid communication with stabilizer port 322 and is in fluid communication with the atmosphere 99; and
- elastic balloon 848, which comprises an inflatable portion 850 that is in fluid communication with stabilizer port 322 and is disposed within expandable wall membrane 834.

For some applications, inflatable portion 850 and expandable wall membrane 834 are configured such that:
(a) when inflatable portion 850 is inflated at a first-medium pressure of 15 cm H2O, such as, for example, could be the pressure illustrated in Fig. 12B:
   (i) none of or less than 15% of an entire inflatable-portion outer surface 852 of inflatable portion 850 touches a wall-membrane internal surface 861 of expandable wall membrane 834, and
   (ii) wall-membrane internal surface 861 has a first-medium-pressure area, and
(b) when inflatable portion 850 is inflated at a second-medium pressure of 30 cm H2O, such as, for example, could be the pressure illustrated in Fig. 12C:
   (i) at least 50% of entire inflatable-portion outer surface 852 touches wall-membrane internal surface 861, and
   (ii) wall-membrane internal surface 861 has a second-medium-pressure area that equals at least 120% of the first-medium-pressure area.

For some applications, cuff pressure stabilizer 800 further comprises a substantially rigid chamber 860. For example, substantially rigid chamber 860 may be defined by a housing 810 of cuff pressure stabilizer 800. Expandable wall membrane 834 is disposed within chamber 860. Chamber 860 is shaped and inflatable portion 850 and expandable wall membrane 834 are configured such that when inflatable portion 850 is inflated at the second-medium pressure of 30 cm H2O, none of or less than 20% of an entire wall-membrane outer surface of expandable wall membrane 834 touches a chamber internal surface 854 of chamber 860, such as, for example, could be the pressure illustrated in Fig. 12C.

For some applications, housing 810 is opaque.

For some applications inflatable portion 850 and expandable wall membrane 834 are configured such that:
- when inflatable portion 850 contains a base low-pressure volume V_{B} of air, inflatable portion 850 has a base low pressure of 10 cm H2O, such as, for example, could be the pressure illustrated in Fig. 12A,
- cuff pressure stabilizer 800 is characterized by a pressure-volume curve that represents the pressure in inflatable portion 850 when inflated with different incremental volumes of air beyond the base low-pressure volume V_{B} of air, and
- an average rate of change of the pressure-volume curve over a first pressure interval between 25 and 30 cm H2O is between 0.3 and 1 cm H2O / cc.

For some applications inflatable portion 850 and expandable wall membrane 834 are configured such that that an average rate of change of the pressure-volume curve over a second pressure interval between 40 and 50 cm H2O is between 0.2 and 2 cm H2O / cc.

Typically, chamber 860 is shaped so as to define at least one opening 811 therethrough to the atmosphere 99, in order to maintain air pressure within chamber 860 but outside elastic balloon 848 and expandable wall membrane 834 at approximately atmospheric pressure. In addition, expandable wall membrane 834 is typically shaped so as to define at least one opening 813 therethrough to chamber 860, and via chamber 860 to the atmosphere 99, in order to maintain air pressure within expandable wall membrane 834 but outside elastic balloon 848 at approximately atmospheric pressure, until expandable wall membrane 834 become confined by chamber 860.

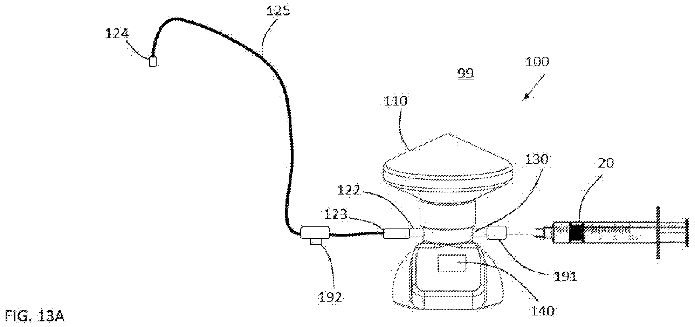

Reference is now made to Figs. 13A-B, which are schematic illustrations of an alternative configuration of cuff pressure stabilizer 100, in accordance with an application of the present invention. Although these features are illustrated with respect to cuff pressure stabilizer 100, they are equally applicable to and may be implemented in combination with the other cuff pressure stabilizers described herein.

In this configuration, cuff pressure stabilizer 100 further comprises an inflation valve 191, which may, for example, comprise an on/off "push-valve" (e.g., conventional ETT inlet valve), or a one-way valve, or another kind of valve. Cuff pressure stabilizer 100 further comprises a switchable pre-inflate valve 193, disposed along a fluid-communication path between stabilizer port 122 and inflation lumen proximal port connector 124, such as along connector tube 125, described hereinabove with reference to Figs. 1A-C. As described hereinabove, inflation lumen proximal port connector 124 that is shaped to form an air-tight seal with inflation lumen proximal port 15 of airway ventilation device 10. Switchable pre-inflate valve 193 is configured to be switchable between a close configuration, in which the valve blocks fluid flow therethrough, and an open configuration, in which the valve allows fluid flow therethrough.

Switchable pre-inflate valve 193 enables a user to choose to inflate and maintain inflatable portion 150 of balloon 148 to a desired pressure before connecting cuff pressure stabilizer 100 (e.g., inflation lumen proximal port connector 124 thereof) to inflation lumen proximal port 15 of airway ventilation device 10.

As shown in Fig. 13A, when switchable pre-inflate valve 193 is in the closed configuration, valve 193 blocks out-flow between balloon 148 and inflation lumen proximal port connector 124. Thus, as indicated in Fig 13A, balloon 148 can be inflated to pressures above 10 cm H2O even when not connected to airway ventilation device 10.

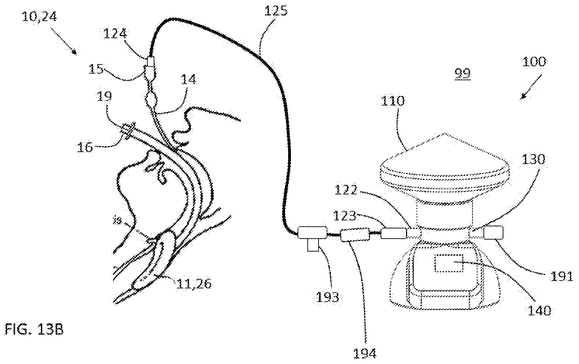

As shown in Fig. 13B, after such inflation and subsequent coupling of inflation lumen proximal port connector 124 to inflation lumen proximal port 15 of airway ventilation device 10, switchable pre-inflate valve 193 is set in the open configuration, in which there is fluid communication between the balloon 148 and inflation lumen proximal port 15 of airway ventilation device 10, and thus with inflatable cuff 11 of airway ventilation device 10.

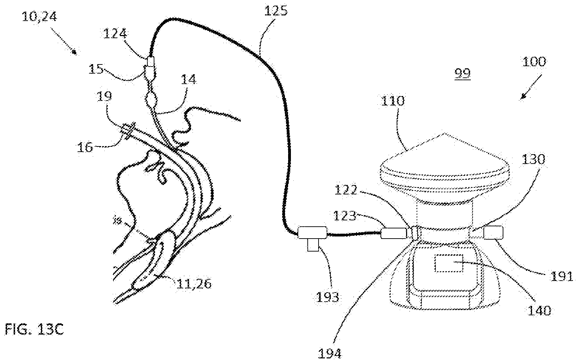

Reference is still made to Fig. 13B, and is additionally made to Fig. 13C. Figs. 13B and 13C are schematic illustrations of additional alternative configurations of cuff pressure stabilizer 100, in accordance with respective applications of the present invention. Although these features are illustrated with respect to cuff pressure stabilizer 100, they are equally applicable to and may be implemented in combination with the other cuff pressure stabilizers described herein.

In these configurations, cuff pressure stabilizer 100 further comprises a flow limiter 194, which is configured to slow the pressure-regulation response time of cuff pressure stabilizer 100. This flow regulation prevents the cuff pressure stabilizer from responding erratically to changes of pressure in inflatable cuff 11 of airway ventilation device within a single ventilation cycle, but rather to regulate only pressure changes that continue over an extended period of time.

For some applications, flow limiter 194 is configured, when exposed to a pressure difference of 5 cm H2O thereacross, to allow air flow therethrough of less than 0.5 cc (e.g., less than 0.3 cc) over a period of 3 seconds (which is the typical half-time of a single breathing cycle), and/or less than 0.167 cc per second (e.g., less than 0.1 cc per second, or less than 0.05 cc per second).

For some applications, in order to provide the flow limitation, flow limiter 194 comprises a tube having a diameter of 0.3 mm and a length of 3 cm.

Flow limiter 194 may be disposed anywhere in the fluid communication path between balloon 148 and inflation lumen proximal port connector 124. For example, flow limiter 194 may be disposed along connector tube 125, as shown in Fig. 13B, or between balloon 148 and stabilizer port 122, such as shown in Fig. 13C.

Reference is made to Figs. 14 and 15A-B, which are schematic illustrations of a cuff pressure stabilizer 900, in accordance with an application of the present invention. Figs. 15A-B are cross-sectional views of Fig. 14 taken along lines XV-XV. Other than as described hereinbelow, cuff pressure stabilizer 900 is generally similar to cuff pressure stabilizer 100, described hereinabove with reference to Figs. 1A-4, and may implement any of the features thereof. Like reference numerals refer to like parts.

Although not shown in Figs. 14 and 15A-B, cuff pressure stabilizer 900 is configured to be used with (a) airway ventilation device 10, which is not a component of cuff pressure stabilizer 900, (b) external inflation source 20, such as a syringe, which is typically not a component of cuff pressure stabilizer 900, and (c) one or more connector tubes, such as described hereinabove with reference to Figs. 1A-C, which are optionally a component of cuff pressure stabilizer 900 (and may be removably or permanently coupled to cuff pressure stabilizer 900). Cuff pressure stabilizer 900 typically comprises stabilizer port 122, which is in fluid communication with an elastic balloon 948, and is configured to be coupled to the one or more connector tubes, such as described hereinabove with reference to Figs. 1A-C. For some applications, cuff pressure stabilizer 900 comprises inflation inlet port 130, which is in fluid communication with elastic balloon 948, such as described hereinabove with reference to Figs. 1A and 1C; for other applications, cuff pressure stabilizer 900 further comprises inlet junction 131, which couples in fluid communication: connector tube 125, an inflation inlet port 132, first connector tube 133, and stabilizer port 122, such as described hereinabove with reference to Fig. 1B.

Cuff pressure stabilizer 900 comprises:
- stabilizer port 122, described hereinabove, which is configured to be coupled in fluid communication with inflation lumen proximal port 15 of airway ventilation device 10;
- a protective housing 910; and
- elastic balloon 948, which is in fluid communication with stabilizer port 122, and which is arranged such that an inflatable portion 950 of balloon 948 is disposed inside protective housing 910 (balloon 948 may include other portions, such as the neck thereof, that are not inflatable because they are constrained from inflating, e.g., by the casing of cuff pressure stabilizer 900).

Protective housing 910 is typically more rigid than elastic balloon 948. For example, protective housing 910 may have a durometer hardness that is at least 3 times (e.g., at least 5 times) greater than a durometer hardness of elastic balloon 948. For example, the durometer hardness may be measured in Shore, such as Shore A, or another scale.

For some applications, protective housing 910 is substantially rigid. As used in the present application, including in the claims and the Inventive Concepts, "substantially rigid," when referring to protective housing 910, means that the protective housing, when disposed in atmosphere 99, does not materially deform at least when the pressure in balloon 948 is between 0 and 120 cm H2O.

Although protective housing 910 is shown as generally right cylindrical, it may alternatively have another tubular shape, such as an elliptical cylinder or a rectangular tube.

For some applications, protective housing 910 is opaque.

Inflatable portion 950 of balloon 948 is shaped so as to define an inflation inlet 914 that is in fluid communication with stabilizer port 122, such as shown in Fig. 15A, and a proximal surface of protective housing 910 is typically shaped so as to define an inflation opening 912 aligned with inflation inlet 914, such that inflatable portion 950 of balloon 948 is inflatable via inflation opening 912 of protective housing 910.

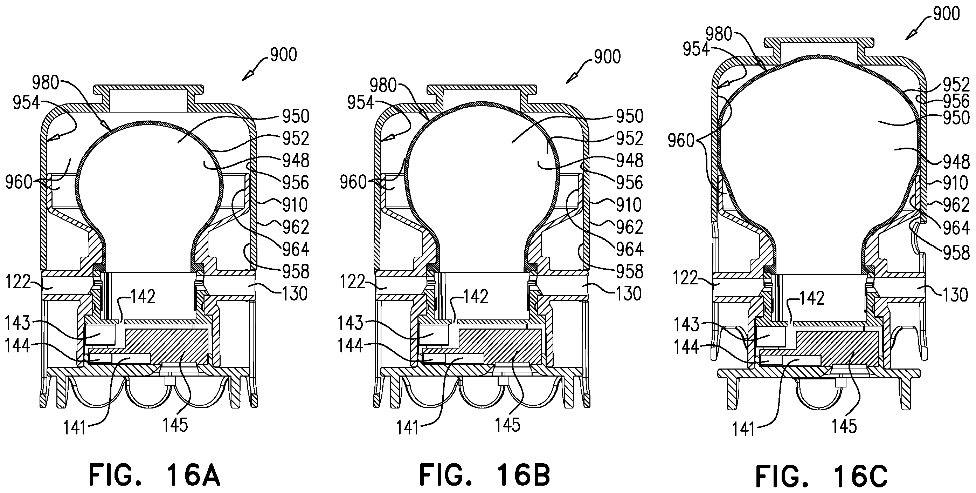

Reference is still made to Figs. 14 and 15A-B, and is additionally made to Figs. 16A-C, which are schematic illustrations of cuff pressure stabilizer 900 with inflatable portion 950 of balloon 948 inflated with different respective volumes, in accordance with an application of the present invention. For some applications, protective housing 910 is shaped and inflatable portion 950 of balloon 948 is configured such that:
- when inflatable portion 950 of balloon 948 contains a base low-pressure volume V_{B} of air, (a) inflatable portion 950 of balloon 948 has a base low pressure of 10 cm H2O, and (b) none of or less than 10% of an outer surface 952 of inflatable portion 950 of balloon 948 touches (i.e., comes in direct physical contact with) an inner surface 954 of protective housing 910 (inflation level not illustrated),
- when inflatable portion 950 of balloon 948 contains a first-medium-pressure volume V₁ of air, (a) inflatable portion 950 of balloon 948 has a first-medium pressure of 15 cm H2O, and (b) none of or less than 15% of an outer surface 952 of inflatable portion 950 of balloon 948 touches (i.e., comes in direct physical contact with) an inner surface 954 of protective housing 910, such as schematically illustrated in Fig. 16A; the first-medium-pressure volume V₁ of air equals the sum of (a) the base low-pressure volume V_{B} of air and (b) a first incremental quantity Q₁ of air of less than 10 cc, and
- when inflatable portion 950 of balloon 948 contains a second-medium-pressure volume V₂ of air, (a) inflatable portion 950 of balloon 948 has a second-medium pressure of 30 cm H2O, and (b) at least 20% of outer surface 952 of inflatable portion 950 of balloon 948 touches a portion of inner surface 954 of protective housing 910, such as schematically illustrated in Fig. 16C; the second-medium-pressure volume V₂ of air equals the sum of (a) the base low-pressure volume V_{B} of air and (b) a second incremental quantity Q₂ of air that is between 10 cc and 50 cc, e.g., between 10 and 40 cc, such as between 10 and 30 cc.

For some applications, when inflatable portion 950 of balloon 848 contains the second-medium-pressure volume V₂ of air, no more than 50% of outer surface 952 of inflatable portion 950 of balloon 948 touches a portion of inner surface 954 of the protective housing 910.

Fig. 16B schematically illustrates inflatable portion 950 of balloon 948 containing another medium-pressure volume of air (greater than first-medium-pressure volume V₁ and less than second-medium-pressure volume V₂), such that inflatable portion 950 of balloon 948 has a medium pressure of 22 cm H2O, and less than 15% of an outer surface 952 of inflatable portion 950 of balloon 948 touches inner surface 954 of protective housing 910.

For example, the above-mentioned base low-pressure volume V_{B} of air may be at least 2 cc, no more than 6 cc, and/or between 2 and 6 cc. For example, the above-mentioned first incremental quantity Q₁ of air may be at least 2 cc, no more than 10 cc (e.g., no more than 7 cc), and/or between 2 and 10 cc, such as between 2 and 7 cc. For example, the above-mentioned second-medium incremental quantity Q₂ of air may be at least 10 cc (e.g., at least 20 cc), no more than 50 cc (e.g., no more than 40 cc), and/or between 10 and 50 cc, such as between 20 and 40 cc.

Alternatively or additionally, for some applications, protective housing 910 is shaped and inflatable portion 950 of balloon 948 is configured such that:
- when inflatable portion 950 of balloon 948 contains a base low-pressure volume V_{B} of air, inflatable portion 950 of balloon 948 has a base low pressure of 10 cm H2O (inflation level not illustrated),
- when inflatable portion 950 of balloon 948 contains a first-medium-pressure volume V₁ of air, (a) inflatable portion 950 of balloon 948 has a first-medium pressure of 15 cm H2O, and (b) none of or less than 15% of an outer surface 952 of inflatable portion 950 of balloon 948 touches (i.e., comes in direct physical contact with) an inner surface 954 of protective housing 910, such as schematically illustrated in Fig. 16A; the first-medium-pressure volume V₁ of air equals the sum of (a) the base low-pressure volume V_{B} of air and (b) a first incremental quantity of air, typically less than 10 cc, and
- when inflatable portion 950 of balloon 948 contains a second-medium-pressure volume V₂ of air, (a) inflatable portion 950 of balloon 948 has a second-medium pressure, and (b) at least 20% of outer surface 952 of inflatable portion 950 of balloon 948 touches a portion of inner surface 954 of protective housing 910, such as schematically illustrated in Fig. 16C; the second-medium-pressure volume V₂ of air equals the sum of (a) the base low-pressure volume V_{B} of air and (b) a second incremental quantity of air that is between 1.1 and 3 times the first incremental quantity of air.

Protective housing 910 is shaped so as to define at least one opening 911 therethrough to the atmosphere 99 (labeled in Fig. 15B), in order to maintain air pressure within protective housing 910 but outside balloon 948 at approximately atmospheric pressure. For some applications in which protective housing 910 comprises moveable portion 962 that is moveably coupled to base portion 964, such as described hereinbelow with reference to Figs. 16A-C, the at least one opening 911 is defined by a small gap (e.g., less than 1 mm in width) between moveable portion 962 and base portion 964; the gap also allows moveable portion 962 to move with respect to base portion 964 with minimal friction. Alternatively or additionally, the at least one opening 911 may be similar to the at least one opening 111 through protective housing 110, described hereinabove with reference to Fig. 2B.

For some applications, protective housing 910 has a volume of at least 20 cc (e.g., at least 30 cc), no more than 100 cc (e.g., no more than 80 cc, such as no more than 60 cc), and/or between 20 and 100 cc, such as between 30 and 80 cc, e.g., between 30 and 60 cc.

Reference is still made to Figs. 15A-B and 16A-C. For some applications, inner surface 954 of protective housing 910 is shaped so as to include one or more cylindrical portions 960. Balloon 948 is arranged such that inflatable portion 950 of balloon 948 is disposed inside protective housing 910, typically such that:
- none or less than 15% of outer surface 952 of inflatable portion 950 of balloon 948 touches the one or more cylindrical portions 960 when inflatable portion 950 of balloon 948 is inflated to a first-medium pressure of 15 cm H2O, such as schematically illustrated in Fig. 16A, and
- at least 20% of outer surface 952 of inflatable portion 950 of balloon 948 touches at least a portion of the one or more cylindrical portions 960 when inflatable portion 950 of balloon 948 is inflated to a second-medium pressure greater than the first-medium pressure, such as schematically illustrated in Fig. 16C.

For some applications, the one or more cylindrical portions 960 of inner surface 954 of protective housing 910 that come into contact with balloon 948 when inflatable portion 950 of balloon 948 is inflated to a medium pressure of 50 cm H2O have an area of at least 10 cm2, no more than 60 cm2, and/or between 10 and 60 cm2.

For some applications, when inflatable portion 950 of balloon 948 contains the second-medium-pressure volume of air, the at least a portion of the one or more cylindrical portions 960 touched by inflatable portion 950 has a length of at least 0.5 cm, such as at least 1 cm, measured along a central longitudinal axis of the one or more cylindrical portions 960.

For some applications, an external surface of protective housing 910 is shaped so as to define one or more loops 961 that protrude outwardly from the external surfaces, for enabling coupling of cuff pressure stabilizer 900 to a conventional pole, rail, hospital wall, or other surface or object. For some applications, cuff pressure stabilizer 900 comprises an attachment strip 963, which passes through the one or more loops 961, and which is configured to be coupleable to a conventional pole, rail, hospital wall, or other surface or object.

Reference is made to Figs. 14, 15A-B, and 16A-C. For some applications, cuff pressure stabilizer 900 may implement some or all of the pressure-sensor-based pressure-sensing, pressure-setting, pressure-display, and/or other user-interface techniques described hereinabove with reference to Figs. 1A-C, 2A-B, and 3A-D.

Reference is still made to Figs. 16A-C. For some applications, protective housing 910 is configured so as to define an internal volume that is expandable from a base internal volume, as shown in Figs. 16A-B, to a greater, expanded internal volume, as shown in Fig. 16C. As used in the present application, including in the claims and the Inventive Concepts, the "internal volume" of protective housing 910 is the space enclosed by protective housing 910, including the volume of inflatable portion 950 of balloon 948, which is within protective housing 910 (the volume of inflatable portion 950 of balloon 948 varies based on pressure, as described herein).

For some applications, protective housing 910 is configured so as to define an inner surface area of inner surface 954 that is expandable from a base inner surface area to a greater, expanded inner surface area, as shown in the transition from Fig. 16B to Fig. 16C.

For some applications, protective housing 910 is configured such that a balloon-exposed portion 956 of inner surface 954 of protective housing 910 is in fluid communication with (but not necessarily touching) outer surface 952 of inflatable portion 950 of balloon 948. (A non-balloon-exposed portion 958 of inner surface 954 of protective housing 910 is not in fluid communication with outer surface 952 of inflatable portion 950 of balloon 948.)

For some applications, protective housing 910 comprises a moveable portion 962 that is moveably coupled to a base portion 964 of cuff pressure stabilizer 900. (Optionally, moveable portion 962 is the entirety of protective housing 910.)

For some applications, protective housing 910 is configured such that balloon-exposed portion 956 of inner surface 954 has a variable surface area. For some applications, the surface area of balloon-exposed portion 956 of inner surface 954 varies based on a relative position of moveable portion 962 with respect to base portion 964.

For some applications, moveable portion 962 of protective housing 910 is moveably coupled to base portion 964 such that the internal volume of the protective housing varies based on a relative position of moveable portion 962 with respect to base portion 964. For some of these applications, moveable portion 962 is axially-slidably coupled to base portion 964 such that the internal volume of protective housing 910 varies based on the relative axial position of moveable portion 962 with respect to base portion 964.

Alternatively or additionally, for some applications, moveable portion 962 of protective housing 910 is moveably coupled to base portion 964 such that the inner surface area of protective housing 910 varies based on a relative position of moveable portion 962 with respect to base portion 964. For some of these applications, moveable portion 962 is axially-slidably coupled to base portion 964 such that the inner surface area of protective housing 910 varies based on the relative axial position of moveable portion 962 with respect to base portion 964.

For some applications, moveable portion 962 is axially-slidably coupled to base portion 964, which may be axially fixed. For example, moveable portion 962 may be disposed radially outward from base portion 964, as shown in the figures. The surface area of balloon-exposed portion 956 of inner surface 954 varies based on the relative axial position of moveable portion 962 with respect to base portion 964.

For some of these applications, protective housing 910 is configured such that inflation of inflatable portion 950 of balloon 948 to below a threshold volume does not cause:
- internal volume of protective housing 910 to expand,
- the inner surface area of protective housing 910 to expand, or
- the surface area of balloon-exposed portion 956 of inner surface 954 to increase.

Protective housing 910 is configured such that inflation of inflatable portion 950 of balloon 948 to beyond the threshold volume causes:
- internal volume of protective housing 910 to expand from the base internal volume to the greater, expanded volume,
- the inner surface area of protective housing 910 to expand from the base inner surface area to the greater, expanded inner surface area, and/or
- the surface area of balloon-exposed portion 956 of inner surface 954 to increase.

For example, inflation of inflatable portion 950 of balloon 948 to beyond the threshold volume may cause outer surface 952 of inflatable portion 950 to push moveable portion 962 with respect to base portion 964, thereby increasing the internal volume of protective housing 910, the inner surface area of protective housing 910, and/or balloon-exposed portion 956 of inner surface 954.

Typically, protective housing 910 is configured such that the surface area of balloon-exposed portion 956 of inner surface 954 is adjustable between minimum and maximum values, and protective housing 910 is provided with balloon-exposed portion 956 of inner surface 954 having the minimum surface area, in order to provide cuff pressure stabilizer 900 with a small form-factor, which increases only as necessary to accommodate higher inflation volumes of inflatable portion 950 of balloon 948 beyond the threshold volume.

For some applications, moveable portion 962 of protective housing 910 includes an accordion-pleated surface that allows for moving of moveable portion with respect to base portion 964 and expansion of the internal volume of protective housing 910 without necessarily increasing the inner surface area of protective housing 910 (configuration not shown).

For some applications, protective housing 910 is configured such that the threshold volume corresponds to a pressure in inflatable portion 950 of balloon 948 of at least 20 cm H2O and/or no more than 40 cm H2O (e.g., no more than 30 cm H2O), such as 25 cm H2O. For some applications, as a result of the value of the threshold volume, balloon-exposed portion 956 of inner surface 954 only increases at pressures that commonly occur when cuff pressure stabilizer 900 is used with a laryngeal mask airway device. As mentioned above, cuffs of tracheal ventilation tubes are typically inflated to 25-30 cm H2O, while cuffs of laryngeal mask airway devices are inflated to 40-60 cm H2O. Thus, at volumes corresponding to pressures less than about 40 cm H2O, such as for use with tracheal ventilation tubes, protective housing 910 has a small form-factor, which may be more convenient for the user.

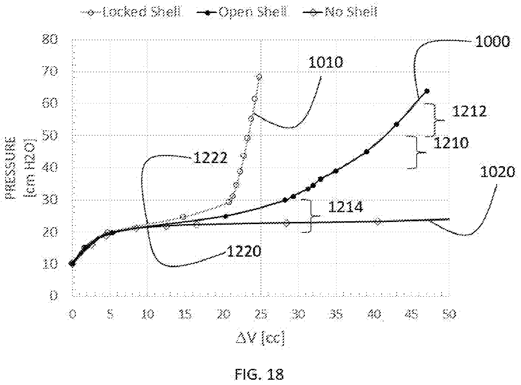

Alternatively, for some applications, balloon-exposed portion 956 of inner surface 954 has a fixed surface area that is large enough to allow inflation of inflatable portion 950 of balloon 948 to at least a volume of 40 cc and/or the volumes shown in pressure-volume curve 1000, described hereinbelow with reference to Fig. 18.

For some applications, protective housing 910 is not shaped so as to define, therethrough to balloon-exposed portion 956 of inner surface 954, an opening having an area of more than 0.5 cm2. Thus, protective housing 910 is not shaped so as to allow access to balloon 948 by a human finger.

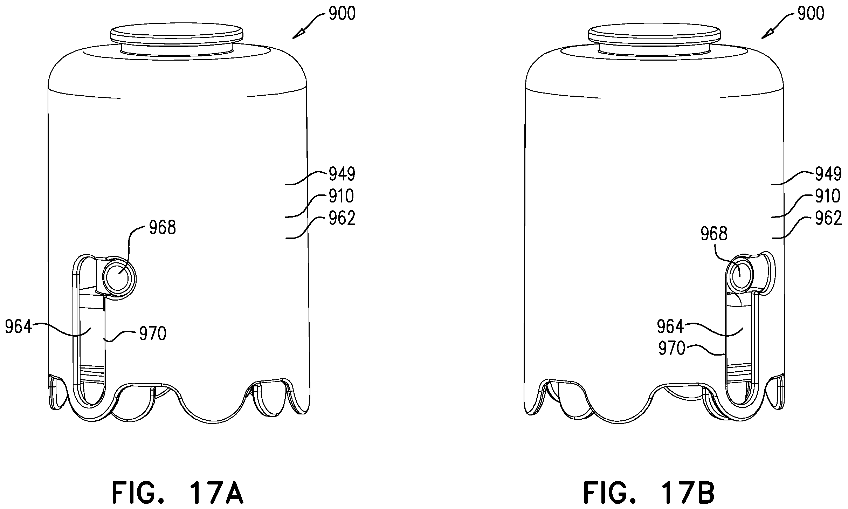

Reference is now made to Figs. 17A-B, which are schematic illustrations of protective housing 910 of cuff pressure stabilizer 900 in locked and unlocked states, in accordance with an application of the present invention. For some applications, cuff pressure stabilizer 900 is configured to assume these locked and unlocked states. For other applications, cuff pressure stabilizer 900 is not configured to assume these locked and unlocked states; for such applications in which balloon-exposed portion 956 of inner surface 954 has the above-described variable surface area, cuff pressure stabilizer 900 is always in the equivalent of the unlocked state.

Cuff pressure stabilizer 900 is configured, when in the locked state, such as shown in Fig. 17A, to prevent moveable portion 962 (and typically the external surface of protective housing 910) from moving (e.g., axially sliding) with respect to base portion 964. As a result, balloon-exposed portion 956 of inner surface 954 has a fixed surface area. This locked state is typically appropriate when cuff pressure stabilizer 900 is used with a tracheal ventilation tube, in which case expansion of the surface area of balloon-exposed portion 956 of inner surface 954 is unnecessary, and it may be desirable to ensure that protective housing 910 retains its small form-factor. Typically, cuff pressure stabilizer 900 will also function in the locked state when coupled to the inflatable cuff of a laryngeal mask airway mask, albeit with less volume available for regulation, but still better than if cuff pressure stabilizer 900 were not coupled to the inflatable cuff of the laryngeal mask airway mask.

Cuff pressure stabilizer 900 is configured, when in the unlocked state, such as shown in Fig. 17B, to allow moveable portion 962 (and typically the external surface of protective housing 910) to move (e.g., axially slide) with respect to base portion 964. As a result, balloon-exposed portion 956 of inner surface 954 has a variable surface area, such as described hereinabove with reference to Figs. 16A-C.

For some applications, a transition between the locked and unlocked states is effected by rotation of moveable portion 962 (and typically the external surface of protective housing 910) with respect to base portion 964. In the locked state, a tab 968 may prevent moving (e.g., axially sliding) of moveable portion 962 with respect to base portion 964. For example, moveable portion 962 (and typically the external surface of protective housing 910) may be shaped so as define an L-shaped slot 970 in which tab 968 may slide both upon rotation of moveable portion 962 (and typically the external surface of protective housing 910) and axially sliding of moveable portion 962 (and typically the external surface of protective housing 910) with respect to base portion 964.

Reference is now made to Fig. 18, which includes pressure-volume curves 1000, 1010, and 1020, in accordance with respective applications of the present invention. Inflatable portion 950 of balloon 948 of cuff pressure stabilizer 900 is characterized by pressure-volume curves 1000 and 1010, which represent the pressure in inflatable portion 950 of balloon 948 when inflated with different incremental volumes of air (ΔV) beyond the base low-pressure volume V_{B} of air corresponding to the base low pressure of 10 cm H2O described hereinabove with reference to Figs. 16A-C. Pressure-volume curves 1000 and 1010 illustrated in Fig. 18 are exemplary pressure-volume curves; a large number of additional pressure-volume curves having the general properties of pressure-volume curves 1000 and 1010 are possible, and are within the scope of the present invention.

For applications in which (a) balloon-exposed portion 956 of inner surface 954 has a variable surface area, such as described hereinabove with reference to Figs. 16A-C, and (b) protective housing 910 has locked and unlocked states, such as described hereinabove with reference to Figs. 17A-B, cuff pressure stabilizer 900 is configured to be characterized by pressure-volume curve 1000 when in the unlocked state described hereinabove with reference to Fig. 17B.

Alternatively, for applications in which (a) balloon-exposed portion 956 of inner surface 954 has a variable surface area, such as described hereinabove with reference to Figs. 16A-C, and (b) protective housing 910 does not have locked and unlocked states, but instead is always in the equivalent of the unlocked state, cuff pressure stabilizer 900 is configured to be characterized by pressure-volume curve 1000.

Further alternatively, for some applications in which balloon-exposed portion 956 of inner surface 954 has a fixed surface area, the surface area is large enough to allow inflation of inflatable portion 950 of balloon 948 to the volumes shown in pressure-volume curve 1000. Cuff pressure stabilizer 900 is thus configured to be characterized by pressure-volume curve 1000.

Still further alternatively, for applications in which (a) balloon-exposed portion 956 of inner surface 954 has a variable surface area, such as described hereinabove with reference to Figs. 16A-C, and (b) protective housing 910 has locked and unlocked states, such as described hereinabove with reference to Figs. 17A-B, cuff pressure stabilizer 900 is configured to be characterized by pressure-volume curve 1010 when in the locked state described hereinabove with reference to Fig. 17A. As mentioned above, this locked state is typically appropriate when cuff pressure stabilizer 900 is used with a tracheal ventilation tube. Typically, cuff pressure stabilizer 900 will also function in the locked state when coupled to the inflatable cuff of a laryngeal mask airway mask, albeit with less volume available for regulation, but still better than if cuff pressure stabilizer 900 were not coupled to the inflatable cuff of the laryngeal mask airway mask.

For some applications, such as shown in Fig. 18, pressure-volume curves 1000 and 1010 do not include a local maximum pressure at any pressure between 20 and 50 cm H2O. By contrast, known pressure-volume curve 202, described hereinabove with reference to Fig. 4, includes a local maximum pressure at about 31 cm H2O (at about 10 cc of incremental air). Alternatively, for other applications (not shown), pressure-volume curves 1000 and 1010 include a local maximum pressure and a local minimum pressure at a greater incremental volume than the local maximum pressure, and (a) a pressure difference between the local maximum pressure and the local minimum pressure is less than 3 cm H2O, e.g., less than 2 cm H2O, and/or (b) a volume difference between the local maximum pressure and the local minimum pressure is less than 40 cc, e.g., less than 30 cc.

For some applications, an average rate of change of pressure-volume curve 1000 over a first pressure interval 1210 between 40 and 50 cm H2O is between 0.5 and 3 cm H2O / cc, such as between 0.5 and 2 cm H2O / cc, e.g., between 0.5 and 1 cm H2O / cc. By contrast, an average rate of change of known pressure-volume curve 202, described hereinabove with reference to Fig. 4, over first pressure interval 210 is about 4 cm H2O / cc.

Alternatively or additionally, for some applications, an average rate of change of pressure-volume curve 1000 over a second pressure interval 1212 between 50 and 60 cm H2O is between 0.5 and 3 cm H2O / cc, such as between 0.5 and 2 cm H2O / cc, e.g., between 0.5 and 1 cm H2O / cc. By contrast, an average rate of change of known pressure-volume curve 202, described hereinabove with reference to Fig. 4, over second pressure interval 212 is about 6 cm H2O / cc. As is known in the mathematical arts, the "average rate of change" is the slope of the secant line joining respective points on the curve at the endpoints of the relevant interval.

Providing these relatively low average rates of change has the effect of stabilizing the pressure in inflatable cuff 28 of laryngeal mask airway device 24. Relatively small increases or decreases in the volume of inflatable cuff 28, for example caused by movement of cuff 28 against the patient's laryngeal inlet, result in corresponding decreases or increases in the volume of inflatable portion 950 of balloon 948. In the relevant typically desired pressure range of laryngeal mask airway cuffs of between 40 and 60 cm H2O, these changes in the volume of inflatable portion 950 have only minimal effect on the pressure in inflatable portion 950, and thus in inflatable cuff 28, because of the elasticity of balloon 948.

Alternatively or additionally, for some applications, an average rate of change of pressure-volume curves 1000 and 1010 over a pressure interval 1214 between 20 and 30 cm H2O is between 0.3 and 5 cm H2O / cc, such as between 0.3 and 3 cm H2O / cc, e.g., between 0.5 and 2 cm H2O / cc, such as between 0.3 and 1 cm H2O. Alternatively or additionally, for some applications, a rate of change of pressure-volume curves 1000 and 1010 at each given pressure over a pressure interval between 22 and 30 cm H2O is between 0.3 and 1 cm H2O / cc. Providing these relatively low average rates of change has the effect of stabilizing the pressure in inflatable cuff 26 of tracheal ventilation tube 22. Relatively small increases or decreases in the volume of inflatable cuff 26, for example caused by movement of cuff 26 in trachea 18, result in corresponding decreases or increases in the volume of inflatable portion 950 of balloon 948. In the relevant typically desired pressure range of tracheal ventilation tube cuffs of between 20 and 30 cm H2O, these changes in the volume of inflatable portion 950 have only minimal effect on the pressure in inflatable portion 950, and thus in inflatable cuff 26, because of the elasticity of balloon 948.

Further alternatively or additionally, for some applications, pressure-volume curve 1000 includes a rising point of inflection 1220 at an inflection-point pressure of between 15 and 30 cm H2O, such as between 15 and 25 cm H2O (e.g., 22 cm H2O), and/or at an incremental volume between 5 and 30 cc, such as between 10 and 20 cc (e.g., 10 cc). For these applications, pressure-volume curve 1000 typically does not include a local maximum pressure at any pressure between 20 and 50 cm H2O. By contrast, known pressure-volume curve 202, described hereinabove with reference to Fig. 4, does not include a rising point of inflection, and does include local maximum and minimum pressures. Still further alternatively or additionally, for some applications, pressure-volume curve 1010 includes a rising point of inflection 1222 at a pressure of between 15 and 30 cm H2O, such as between 15 and 25 cm H2O (e.g., 22 cm H2O), and/or at an incremental volume between 5 and 30 cc, such as between 10 and 20 cc (e.g., 10 cc). For these applications, pressure-volume curve 1010 typically does not include a local maximum pressure at any pressure between 20 and 50 cm H2O.

For some applications, protective housing 910 is shaped and inflatable portion 950 of balloon 948 is configured such that:
- when inflatable portion 950 of balloon 948 is inflated at the base low pressure of 10 cm H2O, at least a base-low-pressure portion 980 of outer surface 952 of inflatable portion 950 of balloon 948 does not touch inner surface 954 of protective housing 910; base-low-pressure portion 980 excludes all portions of outer surface 952 of inflatable portion 950 within 5 mm of inflation inlet 914 of inflatable portion 950 of balloon 948 when inflatable portion 950 has the base low pressure of 10 cm H2O,
- when inflatable portion 950 of balloon 948 is inflated at all pressures greater than 10 cm H2O and less than the inflection-point pressure, none of base-low-pressure portion 980 of outer surface 952 of the inflatable portion of the balloon touches inner surface 954 of protective housing 910, and
- when inflatable portion 950 of balloon 948 is inflated at all pressures at and greater than the inflection-point pressure, base-low-pressure portion 980 of outer surface 952 of inflatable portion 950 of balloon 948 at least partially touches inner surface 954 of protective housing 910.

When inflatable portion 950 of balloon 948 contains the base low-pressure volume V_{B} of air, at least base-low-pressure portion 980 of outer surface 952 of inflatable portion 950 of balloon 948 does not touch inner surface 954 of protective housing 910. At all pressures greater than 10 cm H2O and less than a touching-point pressure, none of base-low-pressure portion 980 of outer surface 952 of inflatable portion 950 of balloon 948 touches inner surface 954 of protective housing 910. Typically, the touching-point pressure is less than 60 cm H2O; for example, the touching-point pressure is greater than 15 cm H2O and less than 30 cm H2O. For some applications, the touching-point pressure corresponds to the inflection-point pressure described above. At all pressures at and greater than the touching-point pressure, base-low-pressure portion 980 of outer surface 952 of inflatable portion 950 of balloon 948 at least partially touches inner surface 954 of protective housing 910.

For some applications, when inflatable portion 950 of balloon 948 is inflated at at all pressures at and greater than the touching-point pressure and less than 60 cm H2O, pressure-volume curve 1000 is convex. Alternatively or additionally, for some applications, at all pressures at and greater than the touching-point pressure and less than 60 cm H2O, pressure-volume curve 1010 is convex.

For some applications:
- when inflatable portion 950 of balloon 948 is inflated at a pressure of 20 cm H2O, a first area of base-low-pressure portion 980 of outer surface 952 of inflatable portion 950 of balloon 948 touches inner surface 954 of protective housing 910,
- when inflatable portion 950 of balloon 948 is inflated at a pressure of 30 cm H2O, a second area of base-low-pressure portion 980 of outer surface 952 of inflatable portion 950 of balloon 948 touches inner surface 954 of protective housing 910, and
- the second area equals at least 3 times the first area.

For some applications, pressure-volume curve 1000 does not include any plateaus. For some applications, pressure-volume curve 1010 does not include any plateaus.

For some applications, inflatable portion 950 of balloon 948 is configured such that, if protective housing 910 were to be removed (or, alternatively, in the absence of protective housing 910), inflatable portion 950 of balloon 948 would be characterized by a removed-protective-housing pressure-volume curve 1020 that represents the pressure in inflatable portion 950 of balloon 948 when inflated with different incremental volumes of air (ΔV) beyond the base low-pressure volume V_{B} of air. It is noted that protective housing 910 is not removed during typical use of cuff pressure stabilizer 900; nevertheless, in these applications, inflatable portion 950 would be characterized by removed-protective-housing pressure-volume curve 1020 if the protective housing were to be removed.

Removed-protective-housing pressure-volume curve 1020 generally provides a good indication of the pressure at which outer surface 952 of inflatable portion 950 of balloon 948 will touch inner surface 954 of protective housing 910 (the inflection point). The flattening of removed-protective-housing pressure-volume curve 1020 means that balloon 948 will expand substantially (and thus reach inner surface 954 of protective housing 910) at this pressure level. For example, in the exemplary pressure-volume curves, this pressure level is about 22 cm H2O. This is also the pressure level about which cuff pressure stabilizer 900 provides maximum volume stability performance. Since the pressure range around 25 cm H2O is of particular importance for the performance of the cuff pressure stabilizer, it may be advantageous to have a balloon for which the pressure-volume curve flattens in the close vicinity of 25 cm H2O.

For some applications, removed-protective-housing pressure-volume curve 1020 includes a local maximum pressure at a pressure (typically, at a single pressure) between 20 and 30 cm H2O. (For example, the illustrated removed-protective-housing pressure-volume curve 1020 includes a very shallow local maximum pressure at about 29 cm H2O when inflated with about 200 cc, beyond the limit of the x-axis shown.)

Reference is again made to Figs. 14-17B. Although the following configurations are described with reference to cuff pressure stabilizer 900, they may also be implemented, *mutatis mutandis,* in the other cuff pressure stabilizers described herein, or in a cuff pressure-sensing device that does not also stabilize or otherwise regulate the pressure in inflatable cuff 11 of airway ventilation device 10; to emphasize the non-essentiality of pressure stabilization in the present configurations, cuff pressure stabilizer 900 is referred to as "pressure-sensing device 900" in the following description.

Pressure-sensing device 900 comprises a connector port 122, analogous to stabilizer port 122 described hereinabove, but referred to as a connector port to emphasize the non-essentiality of pressure stabilization in the present configurations. Connector port 122 is configured to be coupled in fluid communication with inflation lumen proximal port 15 of airway ventilation device 10.

In these configurations, pressure-sensing device 900 further comprises:
- a user-activatable power-ON element;
- pressure sensor 143, which (a) is in fluid communication with connector port 122, and (b) is configured to sense an air pressure;
- a relative-pressure display 140 (shown, by way of example, in Figs. 2A-B);
- circuitry 141, which is electrically coupled to pressure sensor 143; and
- typically, a battery, such as battery power supply 144 shown schematically in Figs. 2B and Figs. 16A-C.

In these configurations, circuitry 141 is configured to be activated by activation of the user-activatable power-ON element to (a) turn on pressure-sensing device 900 (which is typically equivalent to turning on circuitry 141, because circuitry 141 typically controls the other electrical components of the device) and (b) perform a calibration procedure by setting a baseline pressure equal to a current air pressure sensed by pressure sensor 143. In practice, circuitry 141 typically performs the calibration procedure essentially immediately upon activation of the user-activatable power-ON element, such as within one second of activation. In any event, upon activation of the user-activatable power-ON element, circuitry 141 performs the calibration procedure without requiring further user input after the activation.

For some applications, the user-activatable power-ON element comprises a user-activatable button, such as illustrated schematically for turn-ON switch 146 in Fig. 2A. Alternatively, the user-activatable power-ON element may comprise any user-activatable electrical switch, such as a button (e.g., a pushbutton), a toggle switch, a selector switch, a joystick switch, a rocker switch, a knob (e.g., a rotating knob), or an electronic switch.

For some applications, the battery is electrically isolated from circuitry 141 before activation of the user-activatable power-ON element, and the battery, circuitry 141, and the user-activatable power-ON element are arranged such that the activation of the user-activatable power-ON element electrically connects the battery to circuitry 141.

For some applications, the user-activatable power-ON element comprises a battery-isolation tab, which, before activation of the user-activatable power-ON element, is removable disposed electrically between the battery and circuitry 141 so as to electrically isolate the battery from circuitry 141. The user-activatable power-ON element is configured to be activated by removal of the battery-isolation tab from being disposed electrically between the battery and circuitry 141.

Circuitry 141 is configured to, after setting the baseline pressure, periodically drive relative-pressure display 140 to display the difference between (a) the air pressure currently sensed by pressure sensor 143 and (b) the baseline pressure. In other words, the pressure that is generally continuously displayed is the relative pressure within inflatable cuff 11 of airway ventilation device 10 with respect to atmospheric pressure as sensed upon initial activation of the device. (As used in the present application, including in the claims and the Inventive Concepts, "currently" means generally at the current time, and does preclude a small delay between the exact time of sensing and the time of displaying.)

For some applications, pressure-sensing device 900 comprises only a single user-activatable power-ON element, which comprises only a single user-input button.

For some applications, the user-activatable power-ON element is configured not to be de-activatable after the activation thereof, such as to ensure the disposability of the device within the intended time limit of single-patient residence in hospital intensive care.

For some applications, pressure-sensing device 900 does not comprise a user-activatable calibration-reset button other than the user-activatable power-ON element. By contrast, some known electronic pressure-sensing devices include a user-activatable calibration-reset button separate from a user-activatable power-ON element, and is thus activated by the user separately from the user-activatable power-ON element. Providing a user-activatable calibration-reset button separate from a user-activatable power-ON element generally substantially increases the cost of electronic components of the device, which is not desirable in a single-use medical device.

In many electronic pressure-sensing devices, the calibration is performed during manufacture at the factory. The inventors have found that a problem with such factory calibration is that factory-calibrated sensors can degrade to errors of a magnitude of +/- 5 cm H2O, which is very significant for an inflatable cuff 11 of an airway ventilation device 10. The techniques of the present configuration solve this problem by automatically recalibrating the baseline pressure of pressure sensor 143 to the ambient atmospheric pressure when pressure-sensing device 900 is first turned on by the user (before coupling connector port 122 in fluid communication with inflation lumen proximal port 15 of airway ventilation device 10).

For some applications, pressure-sensing device 900 does not comprise any user-activatable elements other than the user-activatable power-ON element.

For some applications, pressure-sensing device 900 further comprises:
- alarm output 151, which is configured to generate a visual and/or audible signal, such as described hereinabove with reference to Figs. 2A-B (alarm output 151 may implement any of the features described hereinabove with reference to Figs. 2A-B); and
- a user-input pressure-threshold-setting interface 153, separate and distinct from the user-activatable power-ON element, such as described hereinabove with reference to Figs. 2A-B (user-input pressure-threshold-setting interface 153 may implement any of the features described hereinabove with reference to Figs. 2A-B).

In these applications, circuitry 141 is configured to set a pressure threshold responsively to an input received from user-input pressure-threshold-setting interface 153, and activate alarm output 151 whenever the pressure sensed by pressure sensor 143 exceeds the pressure threshold by at least a deviation value. Circuitry 141 may implement any of the features described hereinabove with reference to Figs. 2A-B.

Typically, circuitry 141 is configured such that the pressure threshold equals a preset default pressure threshold before circuitry 141 sets the pressure threshold responsively to the input received from the user. For example, the preset default pressure threshold equals between 20 and 30 cm H2O. As a result, if the healthcare worker accidentally couples connector port 122 in fluid communication with inflation lumen proximal port 15 of airway ventilation device 10 before activating he user-activatable power-ON element, the resulting measured pressure of about zero will be substantially less than the preset default pressure threshold, and thus will immediately trigger an alarm.

For some applications, circuitry 141 is configured, upon receiving a set-pressure input from user-input pressure-threshold-setting interface 153, to set the pressure threshold equal to a current air pressure sensed by pressure sensor 143 at a time of receipt of the set-pressure input.

For some applications, circuitry 141 is configured such that the deviation value equals at least 2 cm H2O, such as at least 4 cm H2O, e.g., 5 cm H2O. The deviation value is typically not adjustable by the user, and may be preset as an absolute value, or calculated by circuitry 141, for example as a percentage of the pressure threshold.

For some applications, pressure-sensing device 900 is configured to automatically mechanically and non-electrically stabilize the air pressure in the inflatable cuff without input from pressure sensor 143, when connector port 122 is coupled in fluid communication with inflation lumen proximal port 15, optionally using any of the automatic stabilization techniques described hereinabove.

For some applications, pressure-sensing device 900 further comprises a flow limiter, which is configured to slow a pressure-regulation response time of pressure stabilization provided by pressure-sensing device 900. The flow limiter may implement any of the techniques described hereinabove for flow limiter 194.

To use pressure-sensing device 900, a user (e.g., a healthcare worker), while connector port 122 is open to atmosphere 99 (i.e., not yet connected in fluid communication with inflation lumen proximal port 15 of airway ventilation device 10), activates the user-activatable power-ON element to activate circuitry 141 to (a) turn on pressure-sensing device 900 and (b) perform a calibration procedure by setting a baseline pressure equal to a current air pressure of atmosphere 99 sensed by pressure sensor 143.

Thereafter, the user couples connector port 122 in fluid communication with inflation lumen proximal port 15 of airway ventilation device 10.

By way of example and not limitation, for any of the applications described herein comprising pressure sensor 143, pressure sensor 143 may comprise a digital pressure sensor sold by Bosch Sensortec GmbH (Reutlingen, Germany), such as a BMP280 Digital Pressure Sensor, or by TE Connectivity Ltd. (Schaffhausen, Switzerland), such as a MS5607-02BA03 Barometric Pressure Sensor.

Although cuff pressure stabilizers 100, 300, 600, 700, 800, and 900 have been described as being used with inflatable cuff 11 of airway ventilation device 10, cuff pressure stabilizers 100, 300, 600, 700, 800, and 900 may alternatively be used with other inflatable chambers of other medical devices or non-medical devices. For example, the inflatable chamber may be a Foley catheter balloon, a gastric balloon, a balloon of colonoscope, or a balloon of an endoscope.

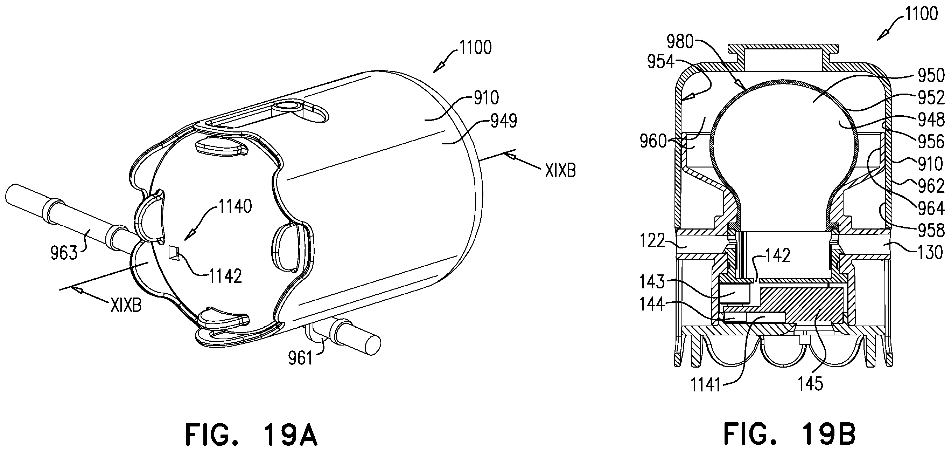

Reference is now made to Figs. 19A-B, which are schematic illustrations of a pressure-sensing device 1100 for use with airway ventilation device 10, in accordance with an application of the present invention. The features of pressure-sensing device 1100 may be implemented in combination with the features of any of the cuff pressure stabilizers described herein, *mutatis mutandis,* or in a cuff pressure-sensing device that does not also stabilize or otherwise regulate the pressure in inflatable cuff 11 of airway ventilation device 10; to emphasize the non-essentiality of pressure stabilization in the present configurations, device 1100 is referred to as "pressure-sensing device 1100," rather than "cuff pressure stabilizer device 1100," in the following description.

Pressure-sensing device 1100 comprises a connector port 122, analogous to stabilizer port 122 described hereinabove, but referred to as a connector port to emphasize the non-essentiality of pressure stabilization in the present configurations. Connector port 122 is configured to be coupled in fluid communication with inflation lumen proximal port 15 of airway ventilation device 10.

Pressure-sensing device 1100 comprises:
- pressure sensor 143, which (a) is in fluid communication with connector port 122, and (b) is configured to sense an air pressure;
- a pressure display 1140, which comprises a multi-color light source 1142; and
- circuitry 1141, which is electrically coupled to pressure sensor 143 and pressure display 1140.

Typically, pressure-sensing device 1100 does not comprise a numerical display (i.e., a display that displays numerical digits) or a textual display (i.e., a display that displays letters, such as letters spelling numbers).

Multi-color light source 1142 of pressure display 1140 is configured to generate at least four different colors having respective spectra, each of the spectra including one or more wavelengths, such as at least five, six, or seven different colors having respective spectra, each of the spectra including one or more wavelengths. Typically, multi-color light source 1142 is configured to generate no more than ten different colors having respective spectra each of the spectra including one or more wavelengths. Multi-color light source 1142 is neither numerical nor textual.

Circuitry 1141 is configured to drive pressure display 1140 to display the air pressure currently sensed by pressure sensor 143, by driving multi-color light source 1142 to generate one of the colors based on predetermined correspondences between the colors and respective preset sets of one or more values of the air pressure. Typically, but not necessarily, circuitry 1141 is configured to periodically drive pressure display 1140 to display the air pressure currently sensed by pressure sensor 143, in which case the generated light electronically flickers (although the flickering may not be perceptible to a human user, e.g., if the light flickers at a rate greater than 20 pulses per second). (As mentioned above, as used in the present application, including in the claims and the Inventive Concepts, "currently" means generally at the current time, and does preclude a small delay between the exact time of sensing and the time of displaying.)

For some applications, multi-color light source 1142 comprises a multi-color LED, such as only a single multi-color LED.

For some applications, multi-color light source 1142 is an RGB (red, green, blue) multi-color light source 1142, e.g., an RGB multi-color LED or another RGB light source. In these applications, multi-color light source 1142 generates the colors by emitting only between one and three colors that mix to generate the four or more colors. In addition, in these applications, the spectra of the colors generated by multi-color light source 1142 of pressure display 1140 create respective visible specific color impressions created by circuitry 1141 tuning the relative power on each of the three RGB channels to create a specific color. In other words, different colors are created not just by the wavelengths of the emitted light, but also by the relative intensities of each of the emitted wavelengths (rather than the total intensity).

Typically, multi-color light source 1142 comprises exactly one picture element, i.e., generates a single dot of whichever color is currently being generated, as opposed to a one-dimensional or two-dimensional array of picture elements, which might be considered graphical. Alternatively, for some applications, multi-color light source 1142 comprises a plurality of picture elements, and circuitry 1141 is configured to drive pressure display 1140 to display the air pressure currently sensed by pressure sensor 143 by driving multi-color light source 1142 to generate, using all of the plurality of picture elements, one of the colors based on the predetermined correspondences between the colors and the respective preset sets of one or more values of the air pressure.

By way of example and not limitation, the following Tables A and B set forth two exemplary correspondences between colors and respective preset sets of one or more air pressures:

**TABLE A**

| Color | Preset set of air pressures (cm H2O) |
|---|---|
| Yellow | < 20 |
| Purple | 20 - 23 |
| Light blue | 23 - 26 |
| White | 26 - 29 |
| Dark blue | 29 - 32 |
| Red | > 32 |

**TABLE B**

| Color | Preset set of air pressures (cm H2O) |
|---|---|
| Red | < 20 |
| Yellow | 20 - 22 |
| Light blue | 22 - 25 |
| White | 25 - 28 |
| Purple | 28 - 31 |
| Red | > 31 |

(For example, for borders between the ranges (such as the exemplary values provided in Tables A and B), circuitry 1141 may be configured to include the border value within a predetermined one of the bordering color ranges. For example, the high border may be included in the exemplary ranges and the low border may be excluded from the exemplary ranges; e.g., for the values in Table A, a pressure of 23 cm H2O may correspond with the color purple, and a pressure of 32 cm H2O may correspond with the color dark blue.)

It is noted that the preset sets of air pressures are not necessary of uniform range. It also noted that the preset sets of air pressures do not necessarily continuously cover the full range between 20 and 30 cm H2O without gaps.

Typically, a color-pressure key is provided to the healthcare worker using pressure-sensing device 1100, such as on an external surface of the device (e.g., on a sticker or printed on the casing of the device), and/or in a printed IFU (instructions for use) document, and/or otherwise.

For some applications, the correspondences include more colors corresponding to values of the air pressure within an acceptable-pressure range between 20 to 30 cm H2O than corresponding to values of the air pressure within a low-pressure range between 10 and 20 cm H2O. In other words, circuitry 1141 may resolve the air pressure measurements non-uniformly, such that more colors are used to resolve pressures within the acceptable-pressure range between 20 to 30 cm H2O than within the low-pressure range of 10 to 20 cm H2O.

Alternatively or additionally for some applications, the correspondences include more colors corresponding to values of the air pressure within an acceptable-pressure range between 20 to 30 cm H2O than corresponding to values of the air pressure within a high-pressure range between 30 and 40 cm H2O. In other words, circuitry 1141 may resolve the air pressure measurements non-uniformly, such that more colors are used to resolve pressures within the acceptable-pressure range between 20 to 30 cm H2O than within the high-pressure range of 10 to 20 cm H2O.

For some applications, the correspondences include correspondences between at least three of the colors and at least three respective preset sets of one or more values of the air pressure within the acceptable-pressure range of values of the air pressure of between 20 and 30 cm H2O.

For some applications, the correspondences include:
- a correspondence between a first one of the colors (e.g., red) and a first one of the respective preset sets of one or more values of the air pressure, the first preset set including both (a) one or more values of the air pressure less than a lower bound of an acceptable-pressure range of values of the air pressure, and (b) one or more values of the air pressure greater than an upper bound of the acceptable-pressure range of values of the air pressure, the upper bound at least 5 cm H2O greater than the lower bound, and
- correspondences between at least three (e.g., at least four) of the colors other than the first color and at least three respective preset sets of one or more values of the air pressure other than the first preset set, each of the preset sets other than the first preset set including one or more values within the acceptable-pressure range of values of the air pressure.

For example, the lower end of the acceptable-pressure range may be a value selected from the group of values between 17 and 21 H2O, and the upper end of the acceptable-pressure range may be a value selected from the group of valves between 28 and 32 H2O.

For some applications, circuitry 1141 is configured to drive multi-color light source 1142 to (a) perceptibly-constantly generate the first color when the currently-sensed pressure corresponds to the one or more values of the air pressure greater than the upper bound of the acceptable-pressure range of values of the air pressure, and (b) blinkingly generate the first color when the currently-sensed pressure corresponds to the one or more values of the air pressure less than the lower bound of an acceptable-pressure range of values of the air pressure. For example, the blinking may be at a rate of at least 2 pulses per second, no more than 10 pulses per second, and/or between 2 and 10 pulses per second, and/or with a pulse duration of at least 10 ms, no more than 500 ms, and/or between 10 and 500 ms. As used in the present application, including in the claims and Inventive Concepts, phrase "perceptibly-constantly" means constantly or at a flicker rate sufficiently fast that a human user cannot sense the flickering, e.g., greater than 20 pulses per second.

For some applications, circuitry 1141 is configured to drive multi-color light source 1142 to (a) blinkingly generate the first color at a first blink rate when the currently-sensed pressure corresponds to the one or more values of the air pressure greater than the upper bound of the acceptable-pressure range of values of the air pressure, and (b) blinkingly generate the first color at a second blink rate when the currently-sensed pressure corresponds to the one or more values of the air pressure less than the lower bound of an acceptable-pressure range of values of the air pressure, the second blink rate different from the first blink rate, e.g., greater than the first blink rate, such as at least twice the first blink rate. For example, the first blink rate may be between 1 and 2 pulses per second and the second blink rate may be between 5 and 10 pulses per second. For example, the first and/or the second blink rates may have a pulse duration of between 10 and 100 ms.

For some applications, one of the preset sets includes at least all values greater than 32 cm H2O.

For some applications, such as for use with laryngeal mask airway cuffs, the lower end of the acceptable-pressure range is a value selected from the group of values between 37 and 41 H2O, and the upper end of the acceptable-pressure range is a value selected from the group of valves between 58 and 62 H2O.

For some applications, one of the preset sets includes at least all values of the air pressure less than 19 cm H2O. For some applications, one of the preset sets includes at least all values of the air pressure less than 19 cm H2O and at least all values of the air pressure greater than 32 cm H2O; as a result, circuitry 1141 is configured to drive multi-color light source 1142 to generate the same one of the colors for at least all values of the air pressure less than 19 cm H2O or greater than 32 cm H2O. For example, red may be the color corresponding to the one of the preset sets.

For some applications, one of the preset sets consists of all values of the air pressure less than 19 cm H2O and all values of the air pressure greater than 32 cm H2O. For example, red may be the color corresponding to the one of the preset sets.

For some applications, one of the preset sets includes at least all values of the air pressure less than a first pressure and at least all values of the air pressure greater than a second pressure, the second pressure greater than the first pressure; as a result, circuitry 1141 is configured to drive multi-color light source 1142 to generate the same one of the colors for at least all values of the air pressure less than the first pressure or greater than the second pressure. For example, red may be the color corresponding to the one of the preset sets.

For some applications, when the colors of the correspondences are ordered according to a low-to-high order of the respective preset sets, the colors are not ordered by the order of the colors of the visible spectrum.

For some applications, none of the colors of the correspondences has a wavelength of between 480 and 550 nm, i.e., is not green, which is not a favored color in medical environments.

As mentioned above, the features of pressure-sensing device 1100 may be implemented in combination with the features of any of the cuff pressure stabilizers described herein, *mutatis mutandis,* For example, as described hereinabove regarding cuff pressure stabilizer 900 with reference to Figs. 14-17B, pressure-sensing device 1100 may further comprise (a) a user-activatable power-ON element, which may have the features described hereinabove, and/or (b) a battery, which may have the features described hereinabove, such as the battery-isolation tab.

Alternatively or additionally, for some applications, the features of pressure-sensing device 1100 are implemented in combination with the features of pressure-sensing device 900, described hereinabove with reference to Figs. 14-17B, *mutatis mutandis,* in which case:
- pressure display 1140 is a relative-pressure display, and
- circuitry 1141 is configured to: (i) be activated by activation of the user-activatable power-ON element to (a) turn on the pressure-sensing device and (b) perform a calibration procedure by setting a baseline pressure equal to a current air pressure sensed by the pressure sensor, and (ii) after setting the baseline pressure, periodically drive the relative-pressure display to display the difference between (a) the air pressure currently sensed by the pressure sensor and (b) the baseline pressure.

In this combination of features, the "air pressure" in the description above of pressure-sensing device 1100 is replaced with the "difference" described immediately above. Other features of pressure-sensing device 900, described hereinabove with reference to Figs. 14-17B, may also be implemented, *mutatis mutandis.*

In the description and claims of the present application, each of the verbs, "comprise," "include" and "have," and conjugates thereof, are used to indicate that the object or objects of the verb are not necessarily a complete listing of members, components, elements or parts of the subject or subjects of the verb. The articles "a" and "an" are used herein to refer to one or to more than one (i.e., to at least one) of the grammatical object of the article. By way of example, "an element" means one element or more than one element. The term "including" is used herein to mean, and is used interchangeably with, the phrase "including but not limited to." The term "or" is used herein to mean, and is used interchangeably with, the term "and/or," unless context clearly indicates otherwise. The term "such as" is used herein to mean, and is used interchangeably, with the phrase "such as but not limited to."

In addition, the materials, methods, and examples are illustrative only and not intended to be limiting.

For brevity, some explicit combinations of various features are not explicitly illustrated in the figures and/or described. It is now disclosed that any combination of the method or device features disclosed herein can be combined in any manner - including any combination of features - any combination of features can be included in any embodiment and/or omitted from any embodiments.

In an embodiment, techniques and apparatus described in one or more of the following applications are combined with techniques and apparatus described herein: US Provisional Application 62/305,567, filed March 9, 2016; US Provisional Application 62/402,024, filed September 30, 2016; US Provisional Application 62/405,115, filed October 6, 2016; US Provisional Application 62/448,254, filed January 19, 2017; PCT Publication WO 2017/153988 to Zachar et al.; US Patent Application Publication 2019/0046749 to Zachar et al.; US Provisional Application 62/557,996, filed September 13, 2017; US Patent 10,092,719 to Zachar et al.; US Provisional Application 62/632,668, filed February 20, 2018; US Patent 10,286,170 to Zachar et al.; US Provisional Application 62/758,007, filed November 9, 2018; US Provisional Application 62/774,588, filed December 3, 2018; PCT Publication WO 2019/162939 to Zachar et al.; US Provisional Application 62/855,061, filed May 31, 2019; and US Provisional Application 62/889,804, filed August 21, 2019

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. A pressure-sensing device (900, 1100)
for use with an airway ventilation device (10) having an inflatable cuff (11), an inflation lumen (13), and an inflation lumen proximal port (15),
the pressure-sensing device (900,1100) comprising:
a connector port (122), which is configured to be coupled in fluid communication with the inflation lumen proximal port (124) and
an elastic balloon (148), which is in fluid communication with the connector port (122);
a user-activatable power-ON element (146);
a pressure sensor (143), which (a) is in fluid communication with the connector port (122), and (b) is configured to sense an air pressure;
a relative-pressure display (140,1140); and
circuitry (141), which is electrically coupled to the pressure sensor and the relative- pressure display, and is configured to:
be activated by activation of the user-activatable power-ON element (146) to (a) turn on the pressure-sensing device and (b) perform a calibration procedure by setting a baseline pressure equal to a current air pressure sensed by the pressure sensor (143), and
**characterized in that** said circuitry (141) is further configured to
after setting the baseline pressure, periodically drive the relative-pressure display (140,1140) to display the difference between the air pressure currently sensed by the pressure sensor (143) and the baseline pressure
and **in that** the device (900,1100) further comprises a flow limiter (194) connected in the fluid communication path between the balloon (148) and the inflation lumen proximal port connector (124), said flow limiter (194) being configured to slow a pressure-regulation response time of pressure stabilization provided by the pressure-sensing device (900, 1100) .

2. The pressure-sensing device according to claim 1, wherein the relative-pressure display (140,1140) is numerical, and is configured to display the difference as a numeral.

3. The pressure-sensing device according to claim 1, wherein the user-activatable power-ON element (146) is configured not to be de-activatable after the activation thereof.

4. The pressure-sensing device according to any one of claims 1-3,
wherein the pressure-sensing device (900, 1100) further comprises a battery (144), which is electrically isolated from the circuitry before activation of the user-activatable power-ON element (146), and
wherein the battery (144), the circuitry (141), and the user-activatable power-ON element (146) are arranged such that the activation of the user-activatable power-ON element (146) electrically connects the battery (144) to the circuitry (141).

5. The pressure-sensing device according to claim 3, wherein the pressure-sensing device comprises only a single user-activatable power-ON element (146), which comprises only a single user-input button.

6. The pressure-sensing device according to any one of claims 1-3, further comprising: an alarm output, which is configured to generate a visual and/or audible signal; and a user-input pressure-threshold-setting interface, separate and distinct from the user-activatable power-ON element (146),
wherein the circuitry (141) is configured to:
set a pressure threshold responsively to an input received from the user-input pressure-threshold-setting interface, and
activate the alarm output whenever the pressure sensed by the pressure sensor (143) exceeds the pressure threshold by at least a deviation value.

7. The pressure-sensing device according to claim 6, wherein the preset default pressure threshold equals between 20 and 30 cm H2O.

8. The pressure-sensing device according to claim 6, wherein the circuitry (141) is configured, upon receiving a set-pressure input from the user-input pressure-threshold- setting interface, to set the pressure threshold equal to a current air pressure sensed by the pressure sensor (143) at a time of receipt of the set-pressure input.

9. The pressure-sensing device according to claim 6, wherein the circuitry (141) is configured such that the deviation value equals at least 2 cm H2O.

10. The pressure-sensing device according to any one of claims 1-3, wherein the pressure-sensing device (900,1100) is configured to automatically mechanically and non-electrically stabilize the air pressure in the inflatable cuff (11) without input from the pressure sensor (143), when the connector port (122) is coupled in fluid communication with the inflation lumen proximal port (124).

11. A system comprising the pressure-sensing device (900, 1100) according to any one of claims 1-5, wherein the system further comprises a connector tube (125) , which comprises an inflation lumen proximal port connector (124) that is shaped to form an air-tight seal with the inflation lumen proximal port (15) , wherein the inflation lumen proximal port connector (124) comprises a male conical fitting with a taper.

12. The system according to claim 11, wherein the taper is a 6% taper.

13. A system comprising the pressure-sensing device (900, 1100) according to any one of claims 1-3, wherein the system further comprises the airway ventilation device (10).

14. The system according to claim 13, wherein the airway ventilation device (10) comprises a tracheal ventilation tube.

15. - The system according to claim 13, wherein the airway ventilation device comprises a laryngeal mask airway device

## Patentansprüche

1. Druckerfassungsvorrichtung (900, 1100)
zur Verwendung mit einer Atemwegbeatmungsvorrichtung (10), die eine aufblasbare Manschette (11), ein Aufblaslumen (13) und einen proximalen Anschluss (15) des Aufblaslumens aufweist,
wobei die Druckerfassungsvorrichtung (900, 1100) umfasst:
einen Anschlussport (122), der ausgestaltet ist, in Fluidkommunikation mit dem proximalen Anschlussport (124) des Aufblaslumens gekoppelt zu werden, und
einen elastischen Ballon (148), der in Fluidkommunikation mit dem Anschlussport (122) steht;
ein vom Benutzer aktivierbares Einschalt-Element (146);
einen Drucksensor (143), der (a) in Fluidkommunikation mit dem Anschlussport (122) steht und (b) ausgestaltet ist, einen Luftdruck zu erfassen;
eine Relativdruckanzeige (140, 1140); und
eine Schaltung (141), die elektrisch mit dem Drucksensor und der Relativdruckanzeige gekoppelt ist und ausgestaltet ist, um:
durch Aktivierung des vom Benutzer aktivierbaren Einschalt-Elements (146) aktiviert zu werden, um (a) die Druckerfassungsvorrichtung einzuschalten und (b) ein Kalibrierungsverfahren durchzuführen, indem ein Basisdruck gleich einem aktuellen vom Drucksensor (143) erfassten Luftdruck gesetzt wird,
**dadurch gekennzeichnet, dass** die Schaltung (141) weiterhin ausgestaltet ist, um
nach dem Setzen des Basisdrucks die Relativdruckanzeige (140, 1140) periodisch anzusteuern, um die Differenz zwischen dem Luftdruck, der aktuell vom Drucksensor (143) erfasst wird, und dem Basisdruck anzuzeigen,
und dadurch, dass die Vorrichtung (900, 1100) weiterhin einen Durchflussbegrenzer (194) umfasst, der in dem Fluidkommunikationspfad zwischen dem Ballon (148) und dem proximalen Aufblaslumen-Anschlussportverbinder (124) verbunden ist, wobei der Durchflussbegrenzer (194) ausgestaltet ist, die Druckregulierungs-Ansprechzeit der durch die Druckerfassungsvorrichtung (900, 1100) bereitgestellten Druckstabilisierung zu verlangsamen.

2. Druckerfassungsvorrichtung nach Anspruch 1, wobei die Relativdruckanzeige (140, 1140) numerisch ist und ausgestaltet ist, die Differenz als Zahl anzuzeigen.

3. Druckerfassungsvorrichtung nach Anspruch 1, wobei das vom Benutzer aktivierbare Einschalt-Element (146) ausgestaltet ist, nach seiner Aktivierung nicht deaktivierbar zu sein.

4. Druckerfassungsvorrichtung nach einem der Ansprüche 1-3, wobei die Druckerfassungsvorrichtung (900, 1100) weiterhin eine Batterie (144) umfasst, die elektrisch von der Schaltung isoliert ist, bevor das vom Benutzer aktivierbare Einschalt-Element (146) aktiviert wird, und wobei die Batterie (144), die Schaltung (141) und das vom Benutzer aktivierbare Einschalt-Element (146) so angeordnet sind, dass die Aktivierung des vom Benutzer aktivierbaren Einschalt-Elements (146) die Batterie (144) elektrisch mit der Schaltung (141) verbindet.

5. Druckerfassungsvorrichtung nach Anspruch 3, wobei die Druckerfassungsvorrichtung nur ein einziges vom Benutzer aktivierbares Einschalt-Element (146) umfasst, das nur einen einzigen Benutzereingabe-Knopf umfasst.

6. Druckerfassungsvorrichtung nach einem der Ansprüche 1-3, weiterhin umfassend:
einen Alarm-Ausgang, der ausgestaltet ist, ein visuelles und/oder akustisches Signal zu erzeugen; und
eine Benutzereingabe-Druckschwellenwert-Einstellungsschnittstelle, die getrennt und verschieden von dem vom Benutzer aktivierbaren Einschalt-Element (146) ist,
wobei die Schaltung (141) ausgestaltet ist, um:
einen Druckschwellenwert als Reaktion auf eine Eingabe, die von der Benutzereingabe-Druckschwellenwert-Einstellungsschnittstelle erhalten wird, zu setzen, und
den Alarm-Ausgang zu aktivieren, wann immer der vom Drucksensor (143) erfasste Druck den Druckschwellenwert um mindestens einen Abweichungswert überschreitet.

7. Druckerfassungsvorrichtung nach Anspruch 6, wobei der voreingestellte Standard-Druckschwellenwert zwischen 20 und 30 cm H₂O beträgt.

8. Druckerfassungsvorrichtung nach Anspruch 6, wobei die Schaltung (141) ausgestaltet ist, den Druckschwellenwert gleich einem aktuellen vom Drucksensor (143) zum Zeitpunkt des Empfangs der Druckeinstellungs-Eingabe erfassten Luftdruck zu setzen.

9. Druckerfassungsvorrichtung nach Anspruch 6, wobei die Schaltung (141) so ausgestaltet ist, dass der Abweichungswert mindestens 2 cm H₂O beträgt.

10. Druckerfassungsvorrichtung nach einem der Ansprüche 1-3, wobei die Druckerfassungsvorrichtung (900, 1100) ausgestaltet ist, den Luftdruck in der aufblasbaren Manschette (11) automatisch mechanisch und nicht-elektrisch zu stabilisieren, ohne Eingaben vom Drucksensor (143), wenn der Anschlussport (122) in Fluidkommunikation mit dem proximalen Anschlussport (124) gekoppelt ist.

11. System, umfassend die Druckerfassungsvorrichtung (900, 1100) nach einem der Ansprüche 1-5, wobei das System weiterhin einen Verbindungsschlauch (125) umfasst, der einen proximalen Aufblaslumen-Anschlussportverbinder (124) umfasst, der ausgestaltet ist, einen luftdichten Abschluss mit dem proximalen Anschlussport (15) des Aufblaslumens zu bilden, wobei der Anschlussportverbinder (124) eine männliche konische Passform mit einer Konizität umfasst.

12. System nach Anspruch 11, wobei die Konizität eine 6-%-Konizität ist.

13. System, umfassend die Druckerfassungsvorrichtung (900, 1100) nach einem der Ansprüche 1-3, wobei das System weiterhin die Atemwegbeatmungsvorrichtung (10) umfasst.

14. System nach Anspruch 13, wobei die Atemwegbeatmungsvorrichtung (10) einen trachealen Beatmungstubus umfasst.

15. System nach Anspruch 13, wobei die Atemwegbeatmungsvorrichtung eine Larynxmaske umfasst.

## Revendications

1. Dispositif de détection de pression (900, 1100)
à utiliser avec un dispositif de ventilation des voies respiratoires (10) comportant un manchon gonflable (11), une lumière de gonflage (13) et un port proximal de lumière de gonflage (15),
le dispositif de détection de pression (900, 1100) comprenant :
un port de raccordement (122), qui est configuré pour être couplé en communication fluidique avec le port proximal de lumière de gonflage (124) et
un ballonet élastique (148), qui est en communication fluidique avec le port de raccordement (122) ;
un élément de mise sous tension (146) activable par l'utilisateur ;
un capteur de pression (143) qui (a) est en communication fluidique avec le port de raccordement (122) et (b) est configuré pour détecter une pression d'air ;
un affichage de pression relative (140, 1140) ; et
un circuit (141), qui est couplé électriquement au capteur de pression et à l'affichage de pression relative, et qui sont configurés pour :
être activés par l'activation de l'élément de mise sous tension (146) activable par l'utilisateur afin (a) de mettre sous tension le dispositif de détection de pression et (b) d'effectuer une procédure d'étalonnage en réglant une pression de référence égale à la pression d'air actuelle détectée par le capteur de pression (143), et
**caractérisé en ce que** ledit circuit (141) est en outre configurés pour,
après avoir réglé la pression de référence, commander périodiquement l'affichage de pression relative (140, 1140) afin d'afficher la différence entre la pression d'air actuellement détectée par le capteur de pression (143) et l.la pression de référence
et **en ce que** le dispositif (900, 1100) comprend en outre un limiteur de débit (194) raccordé dans le chemin de communication fluidique entre le ballonet (148) et le connecteur de port proximal de lumière de gonflage (124), ledit limiteur de débit (194) étant configuré pour ralentir un temps de réponse de régulation de pression du stabilisateur de pression fourni par le dispositif de détection de pression (900, 1100).

2. Dispositif de détection de pression selon la revendication 1, dans lequel l'affichage de pression relative (140, 1140) est numérique et est configuré pour afficher la différence sous forme numérique.

3. Dispositif de détection de pression selon la revendication 1, dans lequel l'élément de mise sous tension (146) activable par l'utilisateur est configuré de manière à ne pas pouvoir être désactivé après son activation.

4. Dispositif de détection de pression selon l'une quelconque des revendications 1 à 3,
dans lequel le dispositif de détection de pression (900, 1100) comprend en outre une batterie (144), qui est isolée électriquement du circuit avant l'activation de l'élément de mise sous tension (146) activable par l'utilisateur, et
dans lequel la batterie (144), le circuit (141) et l'élément de mise sous tension (146) activable par l'utilisateur sont agencés de telle sorte que l'activation de l'élément de mise sous tension (146) activable par l'utilisateur relie électriquement la batterie (144) au circuit (141).

5. Dispositif de détection de pression selon la revendication 3, dans lequel le dispositif de détection de pression comprend uniquement un seul élément de mise sous tension (146) activable par l'utilisateur, qui comprend uniquement un seul bouton d'entrée utilisateur.

6. Dispositif de détection de pression selon l'une quelconque des revendications 1 à 3, comprenant en outre : une sortie d'alarme, configurée pour générer un signal visuel et/ou sonore ; et une interface de réglage du seuil de pression à entrée utilisateur, séparée et distincte de l'élément de mise sous tension (146) activable par l'utilisateur,
dans lequel le circuit (141) est configuré pour :
établir un seuil de pression en réponse à une entrée reçue de l'interface de réglage du seuil de pression à entrée utilisateur, et
activer la sortie d'alarme chaque fois que la pression détectée par le capteur de pression (143) dépasse le seuil de pression d'au moins une valeur d'écart.

7. Dispositif de détection de pression selon la revendication 6, dans lequel le seuil de pression par défaut prédéfini est compris entre 20 et 30 cm H₂O.

8. Dispositif de détection de pression selon la revendication 6, dans lequel le circuit (141) est configuré, à la réception d'une entrée de pression de consigne provenant de l'interface de réglage du seuil de pression à entrée utilisateur, pour établir le seuil de pression égal à une pression d'air actuelle détectée par le capteur de pression (143) au moment de la réception de l'entrée de pression de consigne.

9. Dispositif de détection de pression selon la revendication 6, dans lequel le circuit (141) est configuré de telle sorte que la valeur d'écart soit égale à au moins 2 cm H₂O.

10. Dispositif de détection de pression selon l'une quelconque des revendications 1 à 3, dans lequel le dispositif de détection de pression (900, 1100) est configuré pour stabiliser automatiquement, mécaniquement et non électriquement la pression d'air dans le manchon gonflable (11) sans entrée provenant du capteur de pression (143), lorsque le port de raccordement (122) est couplé en communication fluidique avec le port proximal de lumière de gonflage (124).

11. Système comprenant le dispositif de détection de pression (900, 1100) selon l'une quelconque des revendications 1 à 5, dans lequel le système comprend en outre un tube de raccordement (125), qui comprend un connecteur de port proximal de lumière de gonflage (124) qui est formé pour constituer un joint étanche à l'air avec le port proximal de lumière de gonflage (15), dans lequel le connecteur de port proximal de lumière de gonflage (124) comprend un raccord conique mâle avec une conicité.

12. Système selon la revendication 11, dans lequel la conicité est une conicité de 6 %.

13. Système comprenant le dispositif de détection de pression (900, 1100) selon l'une quelconque des revendications 1 à 3, dans lequel le système comprend en outre le dispositif de ventilation des voies respiratoires (10).

14. Système selon la revendication 13, dans lequel le dispositif de ventilation des voies respiratoires (10) comprend un tube de ventilation trachéale.

15. Système selon la revendication 13, dans lequel le dispositif de ventilation des voies respiratoires comprend un dispositif de voies respiratoires à masque laryngé.
